(19) Europäisches Patentamt
European Patent Office
Office européen des brevets

(11) EP 0 682 027 B1

(12) **EUROPÄISCHE PATENTSCHRIFT**

(45) Veröffentlichungstag und Bekanntmachung des Hinweises auf die Patenterteilung:
**15.10.1997 Patentblatt 1997/42**

(51) Int Cl.[6]: **C07D 487/04**, A61K 31/505

(21) Anmeldenummer: **95810271.7**

(22) Anmeldetag: **25.04.1995**

(54) **Pyrrolopyrimidinderivate mit antiproliferativer Wirkung**

Pyrrolopyrimidine derivatives with antiproliferative action

Dérivés de la pyrrolopyrimidine avec une activité anti-proliférative

(84) Benannte Vertragsstaaten:
**AT BE CH DE DK ES FR GB GR IE IT LI LU MC NL PT SE**

(30) Priorität: **03.05.1994 CH 1385/94**
**30.01.1995 CH 245/95**

(43) Veröffentlichungstag der Anmeldung:
**15.11.1995 Patentblatt 1995/46**

(73) Patentinhaber: **Novartis AG**
**4058 Basel (CH)**

(72) Erfinder:
- **Traxler, Peter, Dr.**
  **CH-4124 Schönenbuch (CH)**
- **Furet, Pascal, Dr.**
  **F-68800 Thann (FR)**
- **Brill, Wolfgang K.D., Dr.**
  **D-79650 Schopfheim (DE)**

(56) Entgegenhaltungen:
**EP-A- 0 566 226** **WO-A-92/20642**
**DE-A- 3 036 390**

- **J. HETEROCYCL. CHEM. (JHTCAD,0022152X);85; VOL.22 (3); PP.859-63, JOERGENSEN A ET AL 'Phosphorus pentoxide in organic synthesis. XX. Synthesis of N-aryl-7H-pyrrolo[2,3-d]pyrimidin-4-amines '**
- **CHIMIE THERAPEUTIQUE, Nr. 6, 1971 Seiten 427-438, MARQUET ET AL. 'Sur une novuelle série d'analogues (...)'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 682 027 B1

Printed by Jouve, 75001 PARIS (FR)

**Beschreibung**

Allgemeine Beschreibung der Erfindung

Die vorliegende Erfindung betrifft ein Verfahren zur Herstellung pharmazeutischer Präparate mit Pyrolopyrimidinen mit einem neuen Verwendungszweck; sowie neue Verbindungen dieses Typs, diese neuen Verbindungen zur Anwendung in einem Verfahren zur diagnostischen oder therapeutischen Behandlung des menschlichen oder tierischen Körpers und Verfahren zur Herstellung dieser Verbindungen.

Hintergrund der Erfindung

Da Tumorerkrankungen eine der Haupttodesursachen in den Industriestaaten sind, werden grösste Anstrengungen unternommen mit dem Ziel, wirksame Mittel und Wege zur Tumorbehandlung zur Verfügung zu stellen. Insbesondere werden aufgrund der Vielzahl und Verschiedenheit möglicher Tumorerkrankungen ständig neue pharmazeutische Verbindungen und Präparate benötigt, die aufgrund ihrer Wirkstoffe zur Behandlung von möglichst vielen oder auch sehr speziellen Tumoren geeignet sind.

Chimie thérapeutique 6, 427-38 (1971) nennt antiproliferative Pyrrolopyrimidine, die sich von den hier vorliegenden Verbindungen strukturell in mindestens einem wichtigen Merkmal unterscheiden.

Überraschend wurde nun gefunden, dass die nachfolgend genannten Verbindungen zur therapeutischen Behandlung von Tumorerkrankungen und anderen proliferativen Erkrankungen, wie Psoriasis, geeignet sind:

Detailierte Beschreibung der Erfindung

Bei den erfindungsgemäss anwendbaren Verbindungen handelt es sich um solche der Formel I

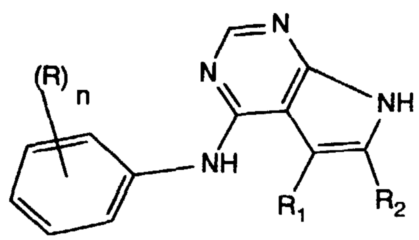

(I)

worin n = 0 bis 5 ist und R für einen oder mehrere Substituenten ausgewählt aus Halogen, Niederalkyl, Trifluormethyl und Niederalkoxy steht; und $R_1$ und $R_2$ unabhängig voneinander jeweils für Niederalkyl oder unsubstituiertes oder durch Halogen, Trifluormethyl, Niederalkyl oder Niederalkoxy substituiertes Phenyl stehen, wobei auch einer der beiden Reste $R_1$ und $R_2$ für Wasserstoff stehen kann, oder gemeinsam eine Alkylenkette mit 2 bis 5 Kohlenstoffatomen bedeuten, welche unsubstituiert oder durch Niederalkyl substituiert ist; oder Salze davon.

Die oben genannte Verwendung gegen Tumoren und weitere proliferative Erkrankungen, wie Psoriasis, ist in keiner Form zu erwarten gewesen. Dasselbe gilt für die unten beschriebene Wirkungsweise über Protein-Kinase-Hemmung.

Die Erfindung betrifft daher die Verwendung dieser Verbindungen zur Herstellung von pharmazeutischen Präparaten zur Behandlung der vor- und nachstehend genannten Erkrankungen.

Die vor- und nachstehend verwendeten Allgemeinbegriffe haben im Rahmen des vorliegenden Textes vorzugsweise die folgenden Bedeutungen:

Das Präfix "Nieder" bezeichnet einen Rest bis und mit maximal 7, insbesondere bis und mit maximal 4, und vor allen Dingen mit 1 oder 2 Kohlenstoffatomen.

"Ferner" steht in der Regel vor Resten oder Bedingungen, die nicht so stark bevorzugt sind wie die jeweils davor genannten.

Die Verbindungen der Formel I können, sofern asymmetrische Kohlenstoffatome vorliegen in Form von Enantiomerengemischen oder (bei 2 oder mehr Asymmetriezentren) Diastereomerengemischen vorliegen, oder in Form der reinen Enantiomeren oder Diastereomeren.

Halogen ist vorzugsweise Fluor, Chlor, Brom oder Iod, insbesondere Fluor, Chlor oder Brom und in erster Linie Brom oder vor allem Chlor.

Niederalkyl ist unverzweigt oder ein- oder mehrfach verzweigt. Bevorzugt ist n-Propyl, Isopropyl, n-Butyl, Isobutyl, sec-Butyl, tert-Butyl, n-Pentyl, Neopentyl, n-Hexyl, insbesondere Ethyl und vor allem Methyl.

Niederalkoxy enthält einen Niederalkylrest wie zuletzt definiert und ist insbesondere n-Propoxy, Isopropoxy, n-Butoxy, Isobutoxy, sec-Butoxy, tert-Butoxy, n-Pentoxy, Neopentoxy, n-Hexoxy, insbesondere Ethoxy und vor allem

Methoxy.

Der unsubstituierte oder durch R ausgewählt aus Halogen, Trifluormethyl, Niederalkyl oder Niederalkoxy substituierte Phenylring in Formel I ist durch einen oder mehrere (maximal n =5), insbesondere durch bis zu 2 (n = 1 oder 2), Substituenten R substituiert, welche in o-, m- oder p-Stellung vorliegen können (wobei stark raumfüllende Substituenten, wie verzweigtes Niederalkyl, z.B. tert-Butoxy, vorzugsweise nicht in o-Stellung vorliegen), insbesondere in m-Stellung; bevorzugt ist (insbesondere, wenn $R_1$ und $R_2$ jeweils Niederalkyl, wie Methyl, bedeuten) der unsubstituierte Phenylrest (n = 0), der in m-Stellung durch Halogen, insbesondere Fluor und vor allem Chlor oder Brom, di- oder insbesondere monosubstituierte Phenylrest (n = 1 oder 2), der in o-, m- oder p-Stellung (insbesondere m-) durch Niederalkoxy, insbesondere Ethoxy und vor allem Methoxy, und/oder der in m-Stellung durch Niederalkyl, insbesondere Methyl, di- oder insbesondere monosubstituierte Phenylrest (n = 1 oder 2); und/oder (wenn $R_1$ und $R_2$ zusammen Alkylen mit 2 bis 5 Kohlenstoffatomen, insbesondere Tetramethylen, bedeuten) der unsubstituierte oder durch Halogen, insbesondere Fluor oder in erster Linie Brom oder Chlor, Trifluormethyl, durch Niederalkoxy, wie Ethoxy oder insbesondere Methoxy, und/oder in m-Stellung durch Niederalkyl, insbesondere Methyl, di- oder insbesondere monosubstituierte Phenylrest (n = 1 oder 2). Besonders bevorzugt sind unsubstituiertes Phenyl (n = 0), m-Chlor-, m-Brom- oder ferner m-Fluor, m,m-Dichlor-, m,m-Dibrom-, m-Methoxy- oder m-Trifluormethyl-phenyl; oder ferner m-Methyl- oder m, p-Dichlor-phenyl (n = 0, 1 oder 2).

Die unabhängig voneinander definierten Reste $R_1$ und $R_2$ können voneinander verschieden oder vorzugsweise gleich sein.

Niederalkyl $R_1$ und $R_2$ hat vorzugsweise die für Niederalkyl oben genannten Bedeutungen, insbesondere die dort als bevorzugt genannten Bedeutungen, und ist in erster Linie Ethyl oder vor allem Methyl.

Unsubstituiertes oder durch Halogen, Trifluormethyl, Niederalkyl oder Niederalkoxy substituiertes Phenyl ist in erster Linie durch einen oder mehrere, insbesondere 1 oder 2, Reste ausgewählt aus Halogen, insbesondere Fluor, Chlor oder Brom, Trifluormethyl, Niederalkyl, insbesondere Methyl oder ferner Ethyl, und Niederalkoxy, wie Methoxy oder ferner Ethoxy, o-, m- oder p-substituiertes Phenyl (wobei vorzugsweise sperrige Substituenten, wie tert-Butoxy, in o-Stellung, sofern nicht herstellbar, ausgeschlossen sind) oder unsubstituiertes Phenyl, und ist insbesondere Phenyl; oder 2-, 3- oder insbesondere 4-Niederalkoxyphenyl oder Diniederalkoxyphenyl, wie 2,5-Diniederalkoxyphenyl, worin Niederalkoxy vorzugsweise Methoxy bedeutet.

Eine gemeinsam aus $R_1$ und $R_2$ gebildete Alkylenkette mit 2 bis 5 Kohlenstoffatomen ist eine Ethylen-, Propylen- oder vorzugsweise eine Pentylen- oder insbesondere Butylenkette, welche durch Niederalkyl, wie oben definiert, insbesondere durch Methyl oder Ethyl, substituiert oder vorzugsweise unsubstituiert vorliegt.

Bevorzugt sind folgende Kombinationen von $R_1$ und $R_2$: $R_1$ bedeutet Niederalkyl und $R_2$ bedeutet Niederalkyl, in erster Linie jeweils Methyl; oder $R_1$ und $R_2$ zusammen bedeuten Pentamethylen oder insbesondere Tetramethylen; oder $R_1$ bedeutet Wasserstoff und $R_2$ bedeutet Phenyl, 2-, 3- oder 4-Niederalkoxyphenyl oder Diniederalkoxyphenyl, wie 2,5-Diniederalkoxyphenyl, wobei Niederalkoxy vorzugsweise jeweils Methoxy bedeutet.

Salze von Verbindungen der Formel I sind, da diese basische Eigenschaften haben, Säureadditionssalze mit organischen oder anorganischen Säuren, insbesondere die pharmazeutisch verwendbaren, nicht-toxischen Salze. Geeignete anorganische Säuren sind beispielsweise Kohlensäure (vorzugsweise in Form der Carbonate oder Bicarbonate); Halogenwasserstoffsäuren, wie Salzsäure; Schwefelsäure; oder Phosphorsäure. Geeignete organische Säuren sind beispielsweise Carbon-, Phosphon-, Sulfon- oder Sulfonaminsäuren, z.B. Essigsäure, Propionsäure, Octansäure, Decansäure, Dodecansäure, Glykolsäure, Milchsäure, 2-Hydroxybuttersäure, Gluconsäure, Glucosemonocarbonsäure, Fumarsäure, Bernsteinsäure, Adipinsäure, Pimelinsäure, Suberinsäure, Azelainsäure, Äpfelsäure, Weinsäure, Zitronensäure, Glucarsäure, Galactarsäure, Aminosäuren, wie Glutaminsäure, Asparaginsäure, N-Methylglycin, Acetylaminoessigsäure, N-Acetylasparagin oder N-Acetylcystin, Brenztraubensäure, Acetessigsäure, Phosphoserin, 2- oder 3-Glycerophosphorsäure, Glucose-6-phosphorsäure, Glucose-1phosphorsäure, Fructose-1 ,6-bisphosphorsäure, Maleinsäure, Hydroxymaleinsäure, Methylmaleinsäure, Cyclohexancarbonsäure, Adamantancarbonsäure, Benzoesäure, Salicylsäure, 1- oder 3-Hydroxynaphthyl-2-carbonsäure, 3,4,5-Trimethoxybenzoesäure, 2-Phenoxybenzoesäure, 2-Acetoxybenzoesäure, 4-Aminosalicylsäure, Phthalsäure, Phenylessigsäure, Mandelsäure, Zimtsäure, Nicotinsäure, Isonicotinsäure, Glucuronsäure, Galacturonsäure, Methan- oder Ethansulfonsäure, 2-Hydroxyethansulfonsäure, Ethan-1,2-disulfonsäure, Benzolsulfonsäure, 2-Naphthalinsulfonsäure, 1,5-Naphthalindisulfonsäure, 2-, 3- oder 4-Methylbenzolsulfonsäure, Methylschwefelsäure, Ethylschwefelsäure, Dodecylschwefelsäure, N-Cyclohexylsulfaminsäure, N-Methyl-, N-Ethyl- oder N-Propylsulfaminsäure, oder andere organische Protonensäuren, wie Ascorbinsäure.

Zur Isolierung oder Reinigung können auch pharmazeutisch ungeeignete Salze, wie z.B. Pikrate oder Perchlorate, Verwendung finden. Zur therapeutischen Anwendung gelangen nur die (bei den entsprechenden Dosen) pharmazeutisch verwendbaren, nicht-toxischen Salze, die deshalb bevorzugt sind.

Die Verbindungen der Formel I, insbesondere die unten genannten neuen erfindungsgemässen Verbindungen, weisen wertvolle pharmakologisch verwendbare Eigenschaften auf. Insbesondere zeigen sie spezifische Hemmwirkungen, die von pharmakologischem Interesse sind. In erster Linie wirken sie als Protein-Tyrosin-Kinase-Hemmer und/oder (ferner) Hemmer von Protein-Serin/Threonin-Kinasen; sie zeigen z.B. eine potente Hemmung der Tyrosinkinaseaktivität des Rezeptors für den epidermalen Wachstumsfaktor (EGF) und der c-erbB2-Kinase. Diese rezeptorspezifischen Enzymaktivitäten spielen eine Schlüsselrolle in der Signalübertragung in einer Vielzahl von Säugetierzellen einschliesslich Humanzellen, insbesondere von Epithelialzellen, Zellen des Immunsystems und Zellen des zentralen und peripheren Nervensystems. Beispielsweise ist die EGF-induzierte Aktivierung der rezeptorassoziierten Protein-Tyrosinkinase (EGF-R-PTK) bei verschiedenen Zelltypen eine Voraussetzung für die Zellteilung und damit die Proliferation der Zellpopulation. Durch die Vermehrung von EGF-Rezeptor-spezifischen Tyrosinkinasehemmern wird somit die Vermehrung der Zellen gehemmt. Analogs gilt für die anderen oben und unten genannten Proteinkinasen.

Die Hemmung der EGF-Rezeptor-spezifischen Protein-Tyrosinkinase (EGF-R-PTK) kann mit bekannten Methoden, z.B. unter Verwendung des rekombinanten intrazellulären Domäne des EGF-Rezeptors (EGF-R ICD; siehe z.B. E. McGlynn et al., Europ. J. Biochem. 207, 265-275 (1992)), nachgewiesen werden. Die Verbindungen der Formel I hemmen die Enzymaktivität gegenüber der Kontrolle ohne Inhibitor zu 50 % (IC50) in 0,001 bis 10 μM, vorzugsweise in 0,001 bis 1 μM Konzentration.

Die Verbindungen der Formel I zeigen ebenfalls im mikromolaren Bereich z.B. auch eine Hemmung des Zellwachstums von EGF-abhängigen Zellinien, etwa der epidermoiden BALB/c-Maus-Keratinocyten-Zellinie (siehe Weissmann, B.A., und Aaronson, S.A., Cell 32, 599 (1983)) oder der A431-Zellinie, die als nützliche Standardquellen von EGF-abhängigen epithelialen Zellen anerkannt sind (siehe Carpenter, G., und Zendegni, J. Anal. Biochem. 153, 279-282 (1985)). In einer bekannten Testmethode (siehe Meyer at al., Int. J. Cancer 43, 851 (1989)) wird die Hemmwirkung der Verbindungen der Formel I in Kürze wie folgt ermittelt: BALB/MK-Zellen (10 000/Mikrotiterplattenloch) werden auf 96-Loch-Mikrotiterplatten transferiert. Die Testverbindungen (gelöst in DMSO) werden in einer Reihe von Konzentrationen (Verdünnungsreihe) zugegeben, so dass die Endkonzentration an DMSO nicht grösser als 1 % (v/v) ist. Nach der Zugabe werden die Platten für drei Tage inkubiert, während derer die Kontrollkulturen ohne Testsubstanz wenigstens drei Zellteilungszyklen durchlaufen können. Das Wachstum der MK-Zellen wird mittels Methylenblau-Färbung gemessen: Nach der Inkubation werden die Zellen mit Glutaraldehyd fixiert, mit Wasser gewaschen und mit 0,05% Methylenblau angefärbt. Nach einem Waschschritt wird der Farbstoff mit 3 % HCl eluiert und die optische Dichte je Vertiefung der Mikrotiterplatte mit einem Titertek multiskan bei 665 nm gemessen. $IC_{50}$-Werte werden durch ein computergestütztes System ermittelt unter Verwendung der Formel:

$$IC_{50} = (OD_{Test} - OD_{Start})/(OD_{Kontrolle} - OD_{Start}) \times 100.$$

Der $IC_{50}$-Wert in diesen Experimenten wird als diejenige Konzentration der jeweiligen Testverbindung angegeben, die eine um 50 % geringere Zellzahl gegenüber der Kontrolle ohne Hemmstoff ergibt. Die Verbindungen der Formel I zeigen Hemmwirkungen im mikromolaren Bereich, z.B. eine $IC_{50}$ von etwa 0,2 bis 20 μM, vorzugsweise von 0,2 bis 10 μM.

Die Verbindungen der Formel I zeigen auch in vivo eine Hemmung des Wachstums von Tumorzellen, wie z.B. durch den nachfolgend beschriebenen Test gezeigt wird: Der Test basiert auf der Hemmung des Wachstums des humanen Epidermoid-Carcinoms A431 (ATCC No. CRL 1555; American Type Culture Collection, Rockville, Maryland, USA; siehe Santon, J.B., et al., Cancer Research 46, 4701-4705 (1986) und Ozawa, S., et al., Int. J. Cancer 40, 706-710 (1987)), welches in weibliche BALB/c-Nacktmäuse (Bomholtgard, Dänemark) transplantiert wird. Dieses Karzinom zeigt ein Wachstum, welches mit dem Ausmass der EGF-Rezeptor-Expression korreliert. Im Experiment werden *in vivo* gezüchtete Tumoren von etwa 1 $cm^3$ Volumen unter sterilen Bedingungen aus Versuchstieren herausoperiert. Diese Tumoren werden zerkleinert und in 10 Volumina (w/v) Phosphat-gepufferter Saline suspendiert. Die Suspension wird s.c. (0,2 ml/Maus in Phosphat-gepufferter Saline) in die linke Flanke der Tiere injiziert. Alternativ können 1 x $10^6$ Zellen aus einer *in-vitro*-Kultur in 0,2 ml Phosphat-gepufferter Saline injiziert werden. Die Behandlung mit Testverbindungen der Formel I wird 5 oder 7 Tage nach der Transplantation begonnen, wenn die Tumoren einen Durchmesser von 4-5 mm erreicht haben. Die jeweilige Wirksubstanz wird (in verschiedenen Dosen bei unterschiedlichen Tiergruppen) einmal täglich während 15 aufeinanderfolgenden p.o oder i.p. Tagen verabreicht (beispielsweise gelöst in Dimethylsulfoxid/®Tween 80 [Polyoxyethylen(20)sorbitan-monooleat; Warenzeichen der ICI Americas, Inc, USA]/0.9 % NaCl in $H_2O$). Das Tumorwachstum wird durch Messung der Durchmesser der Tumoren entlang dreier aufeinander senkrecht stehender Achsen ermittelt. Mit der bekannten Formel $\pi \times L \times D^2/6$ (siehe Evans, B.D., et al., Brit. J. Cancer 45, 466-8 (1982)) werden die Tumorvolumina errechnet. Die Ergebnisse werden als Behandlung/Kontroll-Prozentwerte (T/C x 100 = T/C%) angegeben. Es werden deutliche Hemmungen des Tumorwachstums gefunden, z.B. T/C%-Werte von weniger als 50, was eine deutliche Hemmung des Tumorwachstums bedeutet.

Die Verbindungen der Formel I hemmen neben oder anstelle der EGF-Rezeptor-ProteinTyrosin-Kinase auch andere Protein-Tyrosin-Kinasen, die in die durch trophische Faktoren vermittelte Signalübertragung involviert sind, z.B. die abl-Kinase, Kinasen aus der Familie der src-Kinasen und die c-erbB2-Kinase (HER-2), sowie Serin/Threonin-Kinasen, z.B. die Proteinkinase C oder CDC-Kinasen, welche alle in der Wachtumsregulation und Transformation bei

Säugetierzellen einschliesslich Humanzellen eine Rolle spielen.

Die Hemmung der c-erbB2-Tyrosinkinase (HER-2) kann z.B. analog der für EGF-R-PTK verwendeten Methode bestimmt werden (siehe C. House et al., Europ. J. Biochem. 140, 363-367 (1984)). Die c-erbB2-Kinase kann nach per se bekannten Protokollen isoliert und ihre Aktivität bestimmt werden (siehe T. Akiyama et al., Science 232, 1644 (1986)).

Die Verbindungen der Formel I, welche die Tyrosinkinaseaktivität des Rezeptors für den epidermalen Wachstumsfaktor (EGF) oder ferner der anderen genannten Protein-Tyrosin-Kinasen hemmen, sind daher z.B. bei der Behandlung benigner oder maligner Tumoren nützlich. Sie sind in der Lage, Tumorregression zu bewirken und Tumormetastasierung und das Wachstum von Mikrometastasen zu verhindern. Insbesondere sind sie bei epidermaler Hyperproliferation (Psoriasis), bei der Behandlung von Neoplasien epithelialen Charakters, z.B. Mammakarzinomen, und/oder bei Leukämien (insbesondere chronischmyeloische Leukämie = CML) anwendbar.

Die Verbindungen der Formel I sind zur Behandlung nicht nur am Menschen, sondern auch an anderen Säugetieren geeignet, beispielsweise an kommerziell verwertbaren Tieren, wie Nagetieren, z.B. Mäuse, Kaninchen oder Ratten. Sie können auch als Standards in den oben genannten Versuchen eingesetzt werden, um den Vergleich mit weiteren Verbindungen zu ermöglichen.

Allgemein betrifft die vorliegende Erfindung auch die Verwendung der Verbindungen der Formel I zur Hemmung der genannten Proteinkinasen.

Daneben sind die Verbindungen der Formel I auch diagnostisch einzusetzen; so können beispielsweise aus Säugetieren, wie Menschen, gewonnene proliferierende Zellen, wie Tumorzellen, die auch in Zellkulturen wachsen, dort auf ihre Empfindlichkeit gegenüber Verbindungen der Formel I getestet werden. Damit können Möglichkeiten zur Therapie besser bestimmt werden.

Die erfindungsgemässen Verbindungen können sowohl alleine als auch in Kombination mit anderen pharmakologisch wirksamen Substanzen angewandt werden, z.B. zusammen mit Inhibitoren der Enzyme der Polyaminsynthese, Inhibitoren der Proteinkinase C, Inhibitoren anderer Tyrosinkinasen, Cytokinen, negativen Wachstumsregulatoren, z. B. TGF-β oder IFN-β, Aromatasehemmern, Antiöstrogenen und/oder Cytostatika.

Bei den nachfolgend genannten bevorzugten Erfindungsgegenständen können anstelle allgemeiner Definitionen, soweit sinnvoll und zweckmässig, die eingangs genannten spezifischeren Definitionen verwendet werden.

Bevorzugt werden Verbindungen der Formel I eingesetzt, worin n = 0 bis 2 ist und R für einen Substituenten ausgewählt aus Halogen, in erster Linie Fluor oder insbesondere Chlor oder Brom, Niederalkoxy, wie Methoxy, und ferner aus Trifluormethyl und Niederalkyl, wie Methyl oder Ethyl, steht, wobei die genannten Substituenten vorzugsweise in m-Stellung des Phenylringes vorliegen; und $R_1$ und $R_2$ die genannten Bedeutungen haben, insbesondere unabhängig voneinander jeweils für Niederalkyl, insbesondere Ethyl oder vor allem Methyl, oder unsubstituiertes oder durch Halogen, insbesondere Fluor, Chlor oder Brom; Trifluormethyl; Niederalkyl, wie Methyl oder Ethyl; oder Niederalkoxy, wie Methoxy oder Ethoxy; substituiertes Phenyl stehen, wobei einer der Reste $R_1$ und $R_2$ auch Wasserstoff bedeuten kann, oder gemeinsam eine Alkylenkette mit 4 bis 5 Kohlenstoffatomen bedeuten, welche unsubstituiert oder durch Niederalkyl, wie Methyl oder Ethyl, substituiert ist; oder Salze davon.

Die Erfindung betrifft auch neue Verbindungen der Formel I und deren Einsatz in den vor- und nachstehend genannten Verfahren und pharmazeutischen Präparaten sowie pharmazeutische Präparate mit diesen Verbindungen, wobei es sich insbesondere um die nachfolgend genannten Verbindungen handelt:

Zu diesen Verbindungen der Formel I gehören insbesondere solche, worin n = 1 oder 2, vorzugsweise 1, ist, R in m- (n = 1) oder m,m-Position (n = 2) gebunden ist und aus Fluor und insbesondere Chlor oder Brom; und (ferner) aus Niederalkoxy, insbesondere Methoxy, ausgewählt ist, und die übrigen Reste die bei der Definition der Substituenten in Formel I genannten Bedeutungen haben, insbesondere $R_1$ und $R_2$ unabhängig voneinander jeweils für Niederalkyl, insbesondere Ethyl oder vor allem Methyl, oder unsubstituiertes oder durch Fluor, Chlor oder Brom, Trifluormethyl, Niederalkyl, wie Methyl oder Ethyl, oder einen oder zwei Niederalkoxyreste, wie Methoxy oder Ethoxy, substituiertes Phenyl stehen, wobei einer der beiden Reste $R_1$ und $R_2$ auch Wasserstoff bedeuten kann, oder gemeinsam eine Alkylenkette mit 4 bis 5 Kohlenstoffatomen bedeuten, welche unsubstituiert oder durch Niederalkyl, wie Methyl oder Ethyl, substituiert ist; oder Salze davon.

Besonders bevorzugt sind Verbindungen der Formel I, worin n = 1 ist, R in m-Position gebunden ist und Brom oder insbesondere Chlor bedeutet; und $R_1$ und $R_2$ unabhängig voneinander für Niederalkyl, insbesondere Methyl, oder ferner für Phenyl stehen, oder gemeinsam einen Tetramethylenrest bilden, oder Salze davon.

Besonders bevorzugt sind insbesondere Verbindungen der Formel I, worin n = 1 ist, R in m-Position gebundenes Chlor oder Brom bedeutet und $R_1$ und $R_2$ jeweils Methyl bedeuten, oder Salze davon.

Ganz besonders bevorzugt sind die folgenden Einzelverbindungen oder deren Salze:

1) 4-(m-Chloranilino)-5,6-dimethyl-7H-pyrrolo[2,3-d]pyrimidin,
2) 4-(m-Bromanilino)-5,6-dimethyl-7H-pyrrolo[2,3-d]pyrimidin,
3) 4-(m-Fluoranilino)-5,6-dimethyl-7H-pyrrolo[2,3-d]pyrimidin,
4) 4-(m,m-Dichloranilino)-5,6-dimethyl-7H-pyrrolo[2,3-d]pyrimidin,

5) 4-(m-Methoxyanilino)-5,6-dimethyl-7H-pyrrolo[2,3-d]pyrimidin,
6) 4-(m-Chloranilino)-5,6-tetramethylen-7H-pyrrolo[2,3-d]pyrimidin,
7) 4-(m-Bromanilino)-5,6-tetramethylen-7H-pyrrolo[2,3-d]pyrimidin,
8) 4-(m-Fluoranilino)-5,6-tetramethylen-7H-pyrrolo[2,3-d]pyrimidin,
9)4-(m-Methoxyanilino)-5,6-tetramethylen-7H-pyrrolo[2,3-d]pyrimidin,
10) 4-(m-Chloranilino)-5,6-diphenyl-7H-pyrrolo[2,3-d]pyrimidin,
11) 4-(m-Bromanilino)-5,6-diphenyl-7H-pyrrolo[2,3-d]pyrimidin,
12)4-(m-Chloranilino)-6-(4-methoxyphenyl)-7H-pyrrolo[2,3-d]pyrimidin,
13) 4-(m-Chloranilino)-6-(2,5-dimethoxyphenyl)-7H-pyrrolo[2,3-d]pyrimidin,
14) 4-(m-Chloranilino)-6-(phenyl)-7H-pyrrolo[2,3-d]pyrimidin,
15) 4-(m-Chloranilino)-5-methyl-6-(4-methoxyphenyl)-7H-pyrrolo[2, 3-d]pyrimidin,
16) 4-(m-Chloranilino)-6-(3-methoxyphenyl)-7H-pyrrolo[2,3-d]pyrimidin, und/oder
17) 4-(m-Chloranilino)-6-(2-methoxyphenyl)-7H-pyrrolo[2,3-d]pyrimidin,

wobei von diesen Verbindungen und Salzen diejenigen mit den Nummern 1), 2), 6) und 7) sowie 12), 13), 14), 16), 17) und ferner 15) ganz besonders bevorzugt sind.

Am stärksten bevorzugt ist der Einsatz der oben genannten neuen Verbindungen und ganz besonders der in den Beispielen genannten Verbindungen bei den oben genannten Verwendungen, Verfahren und pharmazeutischen Präparaten als Wirkstoff. Sehr bevorzugt sind die in den Beispielen spezifisch genannten neuen Verbindungen und Salze.

Die Verbindungen der Formel I können in an sich bekannter Weise hergestellt werden (siehe die Deutsche Offenlegungsschrift DE-OS 30 36 390, publiziert am 13. Mai 1982, und Jorgensen, A., et al., J. Heterocycl. Chem. 22, 859 (1985)), indem man z.B.

a) ein Halogen-pyrrolo-pyrimidin der Formel II,

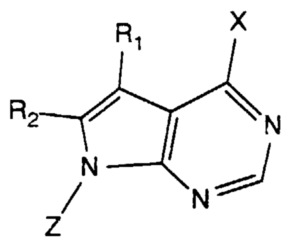

(II)

worin $R_1$ und $R_2$ die für Verbindungen der Formel I genannten Bedeutungen haben, Z für Wasserstoff oder für 1-Arylniederalkan-1-yl steht und X ein Halogenatom bedeutet, mit einem Phenylamin der Formel III,

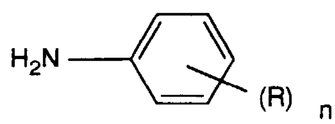

(III)

worin R und n die für Verbindungen der Formel I genannten Bedeutungen haben, umsetzt und, falls Z für 1-Arylniederalkan-1-yl steht, den Rest unter Desalkylierung abspaltet; oder

b) ein Pyrrolo-pyrimidinon der Formel IV,

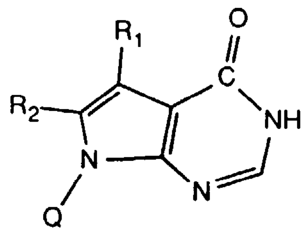

(IV)

worin $R_1$ und $R_2$ die für Verbindungen der Formel I genannten Bedeutungen haben und Q für Wasserstoff oder 1-Aryl-niederalkan-1-yl steht, in Gegenwart eines Dehydratisierungsmittels und eines tertiären Amins mit einem Phenylamin der Formel III, wie zuletzt genannt, umsetzt,

und gewünschtenfalls eine erhaltene freie Verbindung der Formel I in ein Salz umwandelt, ein erhaltenes Salz einer Verbindung der Formel I die freie Verbindung oder in ein anderes Salz umwandelt und/oder ein Gemisch von isomeren Verbindungen der Formel I in die einzelnen Isomeren auftrennt.

Genauere Verfahrensbeschreibung

Bei der nachfolgenden genaueren Beschreibung des Herstellungsverfahrens haben die Reste R, $R_1$ und $R_2$ sowie n die für Verbindungen der Formel I angegebenen Bedeutungen, sofern nichts anderes angegeben ist.

Zu Verfahren a)

In der Verbindung der Formel II ist das Halogen X Brom, Iod oder insbesondere Chlor. 1-Aryl-niederalkan-1-yl Z ist vorzugsweise 1-Phenyl-niederalkyl, insbesondere 1-Phenylethyl oder vor allem Benzyl.

Die Umsetzung zwischen dem Halogenid der Formel II und dem Amin der Formel III findet in geeigneten, inerten polaren Lösungsmitteln statt, insbesondere Alkoholen, z.B. Niederalkanolen, wie Methanol, Propanol oder vor allem Ethanol oder n-Butanol. Die Reaktion findet bei erhöhten Temperaturen statt, vorzugsweise unter Rückflussbedingungen.

Falls Z in der Verbindung der Formel II für 1-Aryl-niederalkan-1-yl steht (d.h., falls eine Verbindung der Formel VIII mit entsprechendem Rest Q als Ausgangsmaterial vorliegt, wie unten definiert), wird dieser Rest in der erhaltenen Vorstufe der Verbindung der Formel I (mit Z anstelle des Wasserstoffatoms am Stickstoff) abgespalten.

Dies geschieht, indem man den 1-Arylniederalkan-1-ylrest Q unter Desalkylierung abspaltet, z.B. durch Behandlung mit Protonensäuren, wie Salzsäure, Phosphorsäuren oder Polyphosphorsäure, bei bevorzugten Temperaturen zwischen 20 und 150 °C und in Ab- oder Anwesenheit von Wasser (dies ist insbesondere die bevorzugte Methode für Z = 1-Phenylethyl); oder vorzugsweise durch Behandlung mit Lewissäuren, insbesondere $AlCl_3$, in aromatischen Lösungsmitteln, insbesondere Benzol und/oder Toluol, bei erhöhter Temperatur, insbesondere unter Rückfluss (dies ist insbesondere die bevorzugte Variante für Z = Benzyl; siehe auch das analoge Verfahren in Chem. Pharm. Bull. 39(5), 1152(1991)).

Zu Verfahren b)

1-Arylniederalkan-1-yl Q in einer Verbindung der Formel IV ist insbesondere 1-Phenylethyl und ferner Benzyl.

Die Verbindung der Formel IV liegt im Tautomeriegleichgewicht (Lactam/Lactim-Form) vor, wobei vermutlich die Lactamform (Formel IV) überwiegt. Die Formel IV wird repräsentativ für die beiden möglichen Gleichgewichtsformen verwendet.

Die Lactimform hat die Formel IVA,

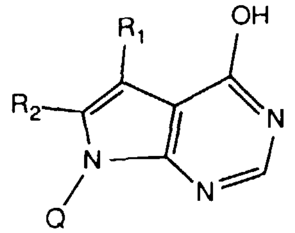

(IVA)

worin die Reste die für Verbindungen der Formel IV genannten Bedeutungen haben. Vorzugsweise steht in einer Verbindung der Formel IV oder IV A der Rest Q für 1-Arylniederalkan-1-yl.

Die Umsetzung zwischen dem Pyrrolo-pyrimidinon der Formel IV und dem Phenylamin der Formel III findet bei erhöhter Temperatur statt, beispielsweise bei 200 bis 250 °C. Als Dehydratisierungsmittel wird insbesondere ein starkes chemisches Dehydratisierungsmittel, vor allem Phosphorpentoxid ($P_4O_{10}$), eingesetzt.

Als tertiäres Amin kommt in erster Linie durch drei Reste, die unabhängig voneinander aus Alkyl, insbesondere Niederalkyl, wie Methyl oder Ethyl, und Cycloalkyl mit 3 bis 7 C-Atomen, insbesondere Cyclohexyl, ausgewählt sind, substituierter Ammoniak in Frage, z.B. N,N-Dimethyl-N-cyclohexylamin, N-Ethyl-N,N-diidopropylamin oder Triethylamin, oder ferner auch Pyridin, N-Methylmorpholin oder 4-Dimethylaminopyridin.

<u>Ausgangsmaterialien</u>:

Die Ausgangsmaterialien der Formeln II und IV können nach folgendem Reaktionsschema (Reaktionsschritte (A) bis (D)) erhalten werden:

Reaktionsschema zur Herstellung von Verbindungen der Formel II und IV

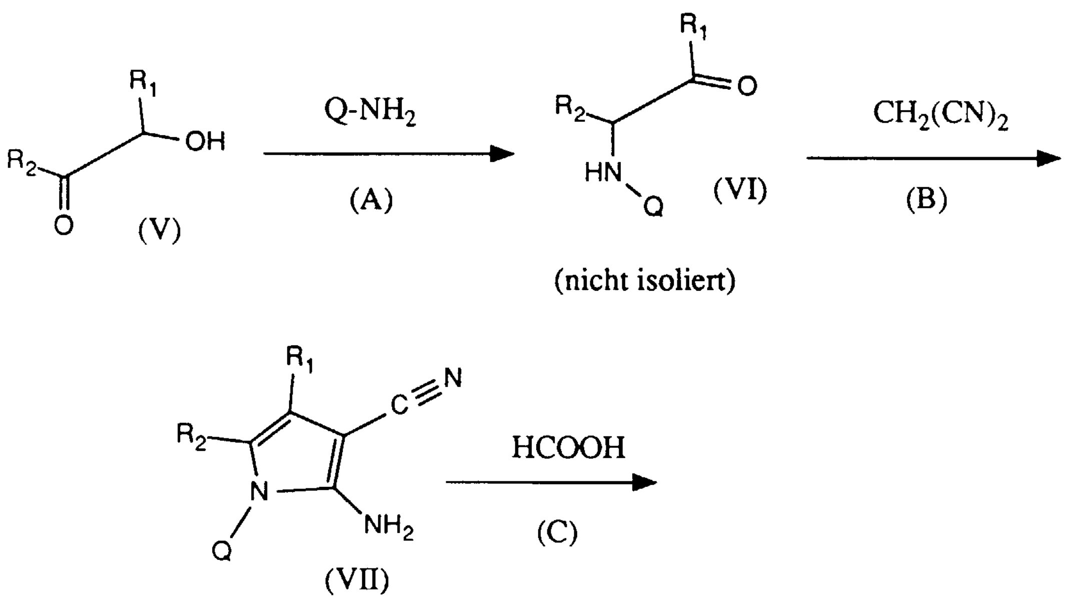

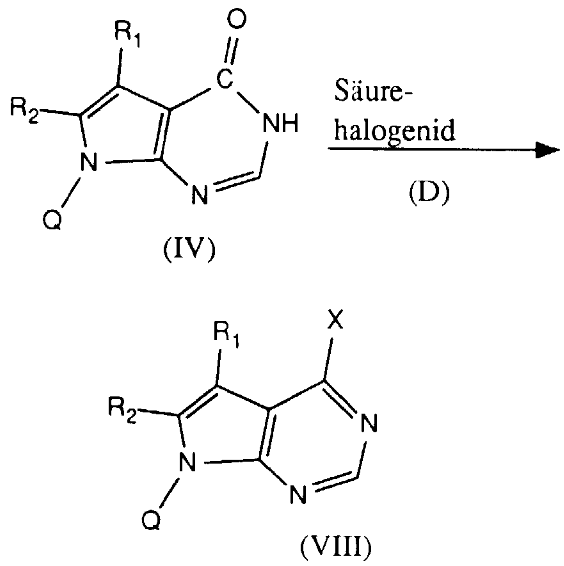


(IV)

(VIII)

Die Verbindung der Formel VIII ist bereits eine Verbindung der Formel II, worin anstelle von Z der Rest Q, welcher für Wasserstoff oder 1-Arylniederalkan-1-yl (insbesondere 1-Phenylniederalkyl, wie Benzyl oder 1-Phenylethyl) steht, vorhanden ist; in den Verbindungen der Formeln IV, V, VI, VII und VIII haben die Reste $R_1$ und $R_2$ die für Verbindungen der Formel I genannten Bedeutungen, während Q (auch in den Stickstoffverbindungen der Formel Q-$NH_2$) für Wasserstoff oder 1-Arylniederalkan-1-yl (insbesondere 1-Phenylniederalkyl, wie Benzyl oder 1-Phenylethyl) steht. In der Verbindung der Formel VIII bedeutet X ein Halogenatom ausgewählt aus Brom, Iod und vor allem Chlor.

Die als Zwischenprodukte verwendeten 1-(Q)-2-Amino-3-cyano-pyrrole der Formel VII lassen sich in Verfahrensschritt (A) und (B) nach an sich bekannten und publizierten Verfahren (siehe z.B. Roth, H.J., und Eger, K., Arch. Pharmaz. 308, 179 (1975)) in guten Ausbeuten darstellen. Die Benzyl-geschützten 4-(X)-Pyrrolo-pyrimidine der Formel VIII (Q = Benzyl; X = Brom, Iod oder insbesondere Chlor)) sind neu und ebenfalls Gegenstand der vorliegenden Erfindung; sie lassen sich nach Verfahren analog den in den Deutschen Offenlegungsschriften DE-OS 28 18 676 (publiziert am 8. Nov. 1979) und DE-OS 30 36 390 (publiziert am 13. Mai 1982) beschriebenen herstellen.

Im Einzelnen erfolgt die Umsetzung (A) mit der Stickstoffverbindung Q-$NH_2$ unter üblichen Kondensationsbedingungen, beispielsweise in Gegenwart von katalytischen Mengen einer starken Säure wie z.B. Salzsäure oder p-Toluolsulfonsäure bei erhöhter Temperatur (vorzugsweise Siedehitze) in einem geeigneten Lösungsmittel, z.B. Benzol oder Toluol, unter Wasserabscheidung zu dem jeweiligen $\alpha$-Aminoketon der Formel VI. Letzteres wird nicht isoliert, sondern sofort mit Malonsäuredinitril in Verfahrensschritt (B) in der Hitze unter weiterer Wasserabscheidung kondensiert, erforerlichenfalls unter Zugabe einer geringen Menge einer Base, wie Piperidin, wobei eine Verbindung der Formel VII erhalten wird.

Die Verbindung der Formel VII wird dann in Verfahrensschritt (C) mit Ameisensäure (welche vorzugsweise im Überschuss gegenüber der Verbindung der Formel VII, beispielsweise im 10 bis 30-molaren Überschuss, eingesetzt wird), gegebenenfalls in Gegenwart inerter Lösungsmittel, wie Dimethylformamid, bei erhöhter Temperatur, z.B. bei Temperaturen zwischen 80°C und Siedetemperatur, umgesetzt, wobei man ein 4-Hydroxy-pyrrolo-pyrimidin der Formel IV erhält.

Die Verbindung der Formel IV kann dann entweder direkt in Verfahren b) (siehe oben) eingesetzt werden, oder mit einem Säurehalogenid zu einer Verbindung der Formel VIII (worin Halogen für Brom, Iod oder insbesondere Chlor steht, also den Rest X bedeutet, wie er in die Verbindung der Formel VIII einzuführen ist) umgesetzt werden (Verfahrensschritt (D)). Als Säurehalogenid sind beispielsweise organische Säurehalogenide, wie Niederalkanoylbromide, -iodide oder vor allem -chloride, oder insbesondere Sulfonsäurebromide, -iodide oder vor allem -chloride, wie p-Toluolsulfonsäurechlorid, oder in erster Linie anorganische Säurebromide, -iodide oder vor allem -chloride, wie $POCl_3$ (besonders bevorzugt), $PCl_5$ oder $SOCl_2$ (für X = Cl), ferner $PBr_5$, $SOBr_2$ (für X = Br) oder $PI_5$ (X = I), geeignet. Die Umsetzung findet statt bei erhöhter Temperatur, z.B. bei Rückflusstemperatur, erforderlichenfalls in Gegenwart eines inerten Lösungsmittels.

Aus der Verbindung der Formel VIII, worin Q einen 1-Arylniederalkan-1-ylrest bedeutet, kann eine Verbindung der Formel II, worin Z Wasserstoff bedeutet, hergestellt werden, indem man den 1-Arylniederalkan-1-ylrest Q unter Dealkylierung abspaltet, z.B. durch Behandlung mit Protonensäuren, wie Salzsäure, Phosphorsäuren oder Polyphosphorsäure, bei bevorzugten Temperaturen zwischen 20 und 150 °C und gegebenenfalls in Gegenwart von Wasser (dies

ist insbesondere die bevorzugte Methode für Q = 1-Phenylethyl); oder vorzugsweise durch Behandlung mit Lewissäuren, insbesondere $AlCl_3$, in einem aromatischen Lösungsmittel, insbesondere in Benzol und/oder Toluol, bei erhöhter Temperatur, insbesondere unter Rückfluss (dies ist insbesondere die bevorzugte Variante für Q = Benzyl; siehe auch das analoge Verfahren in Chem. Pharm. Bull. 39(5), 1152(1991)).

Eine Verbindung der Formel IV, worin $R_1$ für Wasserstoff oder Niederalkyl steht, $R_2$ für unsubstituiertes oder durch Halogen, Trifluormethyl, Alkyl oder Alkoxy substituiertes Phenyl steht und Q für Wasserstoff steht, kann auch nach folgendem Reaktionsschema {Reaktionsschritte E) und F)} gewonnen werden:

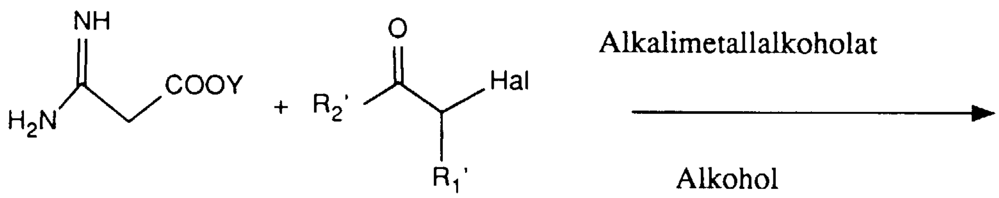


(IX) (X) (E)

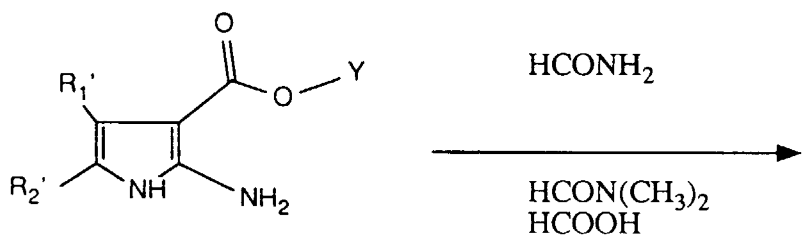


(XI) (F)

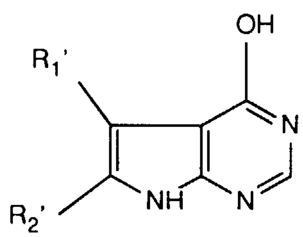
.

(IVB)

Die Verbindung der Formel IVB fällt unter die Definition von Verbindungen der Formel IV (mit analoger Lactim-/Lactamtautomerie, wobei die Formel IVB repräsentativ für beide tautomeren Formen steht) und entspricht einer herzustellenden Verbindung der Formel IV, worin $R_1$ für Wasserstoff oder Niederalkyl steht, $R_2$ für unsubstituiertes oder durch Halogen, Trifluormethyl, Alkyl oder Alkoxy substituiertes Phenyl steht und Q für Wasserstoff steht. In den Verbindungen der Formeln X, XI und IVB bedeutet $R_1'$ Wasserstoff oder Niederalkyl (dem Rest $R_1$ in der herzustellenden Verbindung der Formel IV entsprechend) und $R_2'$ unsubstituiertes oder durch Halogen, Trifluormethyl, Alkyl oder Alkoxy substituiertes Phenyl (dem Rest $R_2$ in der herzustellenden Verbindung der Formel IV entsprechend). Y in Verbindungen der Formeln IX und XI bedeutet einen Alkylrest, wie Niederalkyl, insbesondere Ethyl. Hal in Formel X steht für ein Chlor-, Iod- oder insbesondere Bromatom (auch eine andere Abgangsgruppe, wie Toluolsulfonyloxy oder ein vergleichbarer Sulfonylrest, wäre denkbar).

Im einzelnen erfolgt die Umsetzung (E) in Gegenwart eines Alkalimetallalkoholates, wie Kalium- oder insbesondere Natriumalkoholates, wobei als Alkoholatrest vorzugsweise der Rest eines Alkanols, wie eines Niederalkanols, wie von Methanol oder Ethanol, vorliegt, insbesondere in Gegenwart von Natriumethylat oder ferner Natriummethylat, in Gegenwart des entsprechenden Alkohols, bei bevorzugten Temperaturen zwischen -15 und 50°C, insbesondere zwischen etwa 0 °C und Raumtemperatur, vorzugsweise unter Schutzgas, wie Argon.

Man erhält die entsprechende Verbindung der Formel XI, welche (vorzugsweise nach Reinigung) in Verfahrens-

schritt (F) mit Formamid ($HCONH_2$) [vorzugsweise in Gegenwart geeigneter Lösungsmittel, wie N,N-Dimethylformamid ($HCON(CH_3)_2$] oder anderen N,N-Diniederalkylamiden und in Gegenwart von Ameisensäure umgesetzt wird, bei bevorzugten Temperaturen zwischen 100 und 200 °C, insbesondere zwischen 140 und 160 °C, wobei die Verbindung der Formel IVB erhalten wird.

Bei der Darstellung der Verfahrensschritte (A) bis (D) und (E) bis (F) sind für die Reaktion unwesentliche Nebenprodukte zur Vereinfachung nicht in den oben gezeigten Formeln berücksichtigt.

Amidinoessigsäureester der Formel IX sind bekannt oder nach an sich bekannten Verfahren herstellbar.

$\alpha$-Halogenketone der Formel X sind bekannt, nach an sich bekannten Verfahren herstellbar oder kommerziell erhältlich.

Die Phenylamine der Formel III sind bekannt, kommerziell erhältlich und/oder nach an sich bekannten Verfahren herstellbar.

Allgemeine Verfahrensbedingungen:

Verfahrensgemäss erhältliche freie Verbindungen der Formel I mit salzbildenden Eigenschaften können in an sich bekannter Weise in ihre Salze überführt werden, z.B. durch Behandeln mit Säuren oder geeigneten Derivaten davon, etwa durch Zugabe der betreffenden Säure zu der in einem geeigneten Lösungsmittel, z.B. einem Ether, wie einem cyclischen Ether, inbesondere Dioxan oder vor allem Tetrahydrofuran, gelösten Verbindung der Formel I.

Erfindungsgemäss erhältliche Gemische von Isomeren können in an sich bekannter Weise in die einzelnen Isomeren aufgetrennt werden, Racemate z.B. durch Bilden von Salzen mit optisch reinen salzbildenden Reagentien und Auftrennen des so erhältlichen Diastereomerengemisches, z.B. mittels fraktionierter Kristallisation.

Die oben angeführten Reaktionen können unter an sich bekannten Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln (z.B. Phosphorpentoxid) oder neutralisierenden Agentien, z.B. Basen, insbesondere Stickstoffbasen, wie Triethylamin-hydrochlorid, je nach Art der Reaktion und/oder der Reaktionsteilnehmer bei erniedrigter, normaler oder erhöhter Temperatur, z.B. im Temperaturbereich von etwa -80°C bis etwa 200°C, vorzugsweise von etwa -20°C bis etwa 150°C, z.B. beim Siedepunkt des verwendeten Lösungsmittels, unter atmosphärischem Druck oder in einem geschlossenen Gefäss, gegebenenfalls unter Druck, und/oder in einer inerten Atmosphäre, z.B. unter einer Stickstoffatmosphäre.

Bevorzugt sind die jeweils spezifisch angegebenen Reaktionsbedingungen.

Lösungs- und Verdünnungsmittel sind beispielsweise Wasser, Alkohole, z.B. Niederalkylhydroxide, wie Methanol, Ethanol, Propanol oder insbesondere Butanol, Diole, wie Ethylenglykol, Triole, wie Glycerin, oder Arylalkohole, wie Phenol, Säureamide, z.B. Carbonsäuramide, wie Dimethylformamid, Dimethylacetamid oder 1,3-Dimethyl-3,4,5,6-tetrahydro-2(1H)-pyrimidinon (DMPU), Carbonsäuren, insbesondere Ameisensäure oder Essigsäure, Amide anorganischer Säuren, wie Hexamethylphosphorsäuretriamid, Ether, z.B. cyclische Ether, wie Tetrahydrofuran oder Dioxan, oder acyclische Ether, wie Diethylether oder Ethylenglykoldimethylether, halogenierte Kohlenwasserstoffe, wie Haloniederalkane, z.B. Methylenchlorid oder Chloroform, Ketone, wie Aceton, Nitrile, wie Acetonitril, Säureanhydride, wie Acetanhydrid, Ester, wie Essigsäureethylester, Bisalkansulfine, wie Dimethylsulfoxid, Stickstoffheterocyclen, wie Pyridin, Kohlenwasserstoffe, z.B. Niederalkane, wie Heptan, oder Aromaten, wie Benzol, Toluol oder Xylol(e), oder Gemische dieser Lösungsmittel, wobei die jeweils geeigneten Lösungsmittel für die oben genannten Reaktionen ausgewählt werden können.

Zur Aufarbeitung der erhältlichen Verbindungen der Formel I oder ihrer Salze finden übliche Verfahren Verwendung, beispielsweise Solvolyse von überschüssigen Reagenzien; Umkristallisieren; Chromatographieren, z.B. Verteilungs-, Ionen- oder Gelchromatographie; Verteilung zwischen anorganischer und organischer Lösungsmittelphase; ein- oder mehrfache Extraktion, insbesondere nach Ansäuern oder Erhöhung der Basizität oder des Salzgehaltes; Trocknen über hygroskopischen Salzen; Digerieren; Filtrieren; Waschen; Auflösen; Eindampfen (erforderlichenfalls im Vakuum oder Hochvakuum); Destillation; Kristallisation, beispielsweise von erhaltenen Verbindungen in Ölform oder aus der Mutterlauge, wobei auch mit einem Kristall des Endproduktes angeimpft werden kann; oder eine Kombination zweier oder mehrerer der genannten Aufarbeitungsschritte, die auch wiederholt eingesetzt werden können.

Ausgangsmaterialien und Zwischenprodukte können in reiner Form, beispielsweise nach Aufarbeitung, wie zuletzt erwähnt, in teilweise gereinigter Form oder auch beispielsweise direkt als Rohprodukt verwendet werden.

Infolge der engen Beziehung zwischen den Verbindungen der Formel I in freier Form und in Form von Salzen sind vor- und nachstehend unter den freien Verbindungen bzw. ihren Salzen sinn- und zweckmässig gegebenenfalls auch die entsprechenden Salze bzw. freien Verbindungen zu verstehen, sofern die Verbindungen salzbildende Gruppen enthalten.

Die Verbindungen, einschliesslich ihrer Salze, können auch in Form von Hydraten erhalten werden, oder ihre Kristalle können z.B. das zur Kristallisation verwendete Lösungsmittel einschliessen.

Im Verfahren der vorliegenden Erfindung werden vorzugsweise solche Ausgangsstoffe eingesetzt, die zu den eingangs als besonders wertvoll beschriebenen neuen Verbindungen der Formel I führen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsstoff unter den Reaktionsbedingungen gebildet oder in Form eines Derivates, z.B. eines Salzes davon, verwendet wird.

Pharmazeutische Präparate, deren Herstellung und die erfindungsgemässe Verwendung von Verbindungen der Formel I und Präparaten mit diesen Verbindungen als Wirkstoff

Die vorliegende Erfindung betrifft ebenfalls die Herstellung pharmazeutischer Präparate, die als Wirkstoff eine der Verbindungen der Formel I enthalten und insbesondere zur Behandlung der eingangs genannten Erkrankungen verwendet werden können. Besonders bevorzugt sind Präparate zur enteralen, wie nasalen, bukkalen, rektalen oder insbesondere oralen, sowie zur parenteralen, wie intravenösen, intramuskulären oder subkutanen Verabreichung an Warmblüter, insbesondere Menschen. Die Präparate enthalten den Wirkstoff allein oder vorzugsweise zusammen mit einem pharmazeutisch anwendbaren Trägermaterial. Die Dosierung des Wirkstoffs hängt von der zu behandelnden Krankheit, sowie von Spezies, deren Alter, Gewicht und individuellem Zustand, individuellen pharmakokinetischen Gegebenheiten sowie der Applikationsweise ab.

Die Erfindung betrifft auch pharmazeutische Präparate mit neuen Verbindungen der Formel I zur Anwendung in einem Verfahren zur prophylaktischen oder insbesondere therapeutischen Behandlung des menschlichen oder tierischen Körpers, und ein Verfahren zu deren Herstellung (insbesondere als Mittel zur Tumorbehandlung).

Die Erfindung betrifft auch die Verwendung von Verbindungen der Formel I zur Herstellung von pharmazeutischen Präparaten, die Verbindungen der Formel I als aktive Komponente (Wirkstoff) enthalten.

Bevorzugt ist eine pharmazeutische Zusammensetzung, welche geeignet ist zur Verabreichung an einen Warmblüter, insbesondere Menschen oder kommerziell nutzbaren Säuger, der an einer Erkrankung leidet, die auf eine Hemmung einer Proteinkinase anspricht, beispielsweise Psoriasis oder ein Tumor, umfassend eine zur Hemmung der Proteinkinase wirksame Menge einer Verbindung der Formel I oder eines Salzes davon, falls salzbildende Gruppen vorliegen, zusammen mit mindestens einem pharmazeutisch annehmbaren Trägermaterial.

Eine pharmazeutische Zusammensetzung zur prophylaktischen oder insbesondere therapeutischen Behandlung von Tumorerkrankungen und anderen proliferativen Erkrankungen eines Warmblüters, insbesondere eines Menschen oder eines kommerziell nutzbaren Säugers, der einer derartigen Behandlung bedarf, insbesondere an einer derartigen Erkrankung leidet, enthaltend eine neue Verbindung der Formel I, oder ein pharmazeutisch annehmbares Salz davon, als Wirkstoff in einer prophylaktisch oder insbeosndere therapeutisch gegen die genannten Erkrankungen wirksamen Menge, ist ebenfalls bevorzugt.

Die pharmazeutischen Präparate enthalten von etwa 1 % bis etwa 95 % des Wirkstoffs, wobei einzeldosierte Applikationsformen vorzugsweise von etwa 20 % bis etwa 90 % und nicht-einzeldosierte Applikationsformen vorzugsweise etwa 5 % bis etwa 20 % Wirkstoff aufweisen. Dosiseinheitsformen, sind z.B. Dragées, Tabletten, Ampullen, Vials, Suppositorien oder Kapseln. Weitere Applikationsformen sind z.B. Salben, Crèmes, Pasten, Schäume, Tinkturen, Lippenstifte, Tropfen, Sprays, Dispersionen etc. Beispiele sind Kapseln, enthaltend von etwa 0,05 g bis etwa 1,0 g des Wirkstoffs.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt.

Vorzugsweise verwendet man Lösungen des Wirkstoffes, daneben auch Suspensionen oder Dispersionen, und zwar insbesondere isotonische wässrige Lösungen, Dispersionen oder Suspensionen, wobei diese z. B. bei lyophilisierten Präparaten, welche die Wirksubstanz allein oder zusammen mit einem Trägermaterial, z. B. Mannit, enthalten, vor Gebrauch hergestellt werden können. Die pharmazeutischen Präparate können sterilisiert sein und/oder Hilfsstoffe, z. B. Konservier-, Stabilisier-, Netz- und/oder Emulgiermittel, Löslichkeitsvermittler, Salze zur Regulierung des osmotischen Druckes und/oder Puffer enthalten und werden in an sich bekannter Weise, z. B. mittels konventioneller Lösungs- oder Lyophilisierungsverfahren, hergestellt. Die genannten Lösungen oder Suspensionen können viskositätserhöhende Stoffe, wie Natriumcarboxymethylcellulose, Carboxymethylcellulose, Dextran, Polyvinylpyrrolidon oder Gelatine, enthalten, oder auch Lösungsvermittler, beispielsweise ®Tween 80 [Polyoxyethylen(20)sorbitan-monooleat; Warenzeichen der ICI Americas, Inc, USA].

Suspensionen in Öl enthalten als ölige Komponente die für Injektionszwecke üblichen vegetabilen, synthetischen oder halbsynthetischen Öle. Als solche sind insbesondere flüssige Fettsäureester zu nennen, die als Säurekomponente eine langkettige Fettsäure mit 8-22, insbesondere 12-22, Kohlenstoffatomen, wie z. B. Laurinsäure, Tridecylsäure, Myristinsäure, Pentadecylsäure, Palmitinsäure, Margarinsäure, Stearinsäure, Arachidinsäure, Behensäure oder entsprechende ungesättigte Säuren, wie z. B. Ölsäure, Elaidinsäure, Erucasäure, Brasidinsäure oder Linolsäure, enthalten, gegebenenfalls unter Zusatz von Antioxidantien, wie z. B. Vitamin E, β-Carotin oder 3,5-Di-tert-butyl-4-hydroxytoluol. Die Alkoholkomponente dieser Fettsäureester hat maximal 6 Kohlenstoffatome und ist ein ein- oder mehrwertiger, z. B. ein-, zwei- oder dreiwertiger Alkohol, z. B. Methanol, Ethanol, Propanol, Butanol oder Pentanol oder deren Isomere, vor allem aber Glycol und Glycerin. Als Fettsäureester sind daher beispielsweise zu nennen: Ethyloleat, Isopropylmyristat, Isopropylpalmitat, "Labrafil M 2375" (Polyoxyethylenglycerintrioleat der Firma Gattefossé, Paris), "Labrafil M 1944 CS" (ungesättigte polyglykolisierte Glyceride hergestellt durch eine Alkoholyse von Aprikosenkernöl und konstituiert aus Glyceriden und Polyethylenglykolester; Gattefossé, Frankreich), "Labrasol" (gesättigte polyglykolisierte Glyceride hergestellt durch eine Alkoholyse von TCM und konstituiert aus Glyceriden und Polyethylenglykolester; Gattefossé, Frankreich) und/oder "Miglyol 812" (Triglycerid gesättigter Fettsäuren der Kettenlänge $C_8$ bis $C_{12}$ der Firma Hüls AG, Deutschland), besonders aber vegetabile Öle wie Baumwollsaatöl, Mandelöl, Olivenöl, Ricinusöl, Sesamöl, Sojabohnenöl und vor allem Erdnussöl.

Die Herstellung der Injektionspräparate erfolgt in üblicher Weise unter sterilen Bedingungen, ebenso das Abfüllen beispielsweise in Ampullen oder Vialen sowie das Verschliessen der Behälter.

Beispielsweise kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol, oder Derivate davon.

Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Oral anwendbare pharmazeutische Zusammensetzungen sind ebenfalls Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten flüssigen Hilfsstoffen, wie fetten Oelen, Paraffinöl oder flüssigen Polyethylenglykolen oder Fettsäureestern von Ethylen- oder Propylenglykol, gelöst oder suspendiert, wobei ebenfalls Stabilisatoren und Detergentien, z.B. des Polyoxyethylen-Sorbitan-Fettsäureester-Typs, zugefügt sein können.

Weitere orale Applikationsformen sind z.B. in üblicher Weise bereitete Sirups, die den Wirkstoff z.B. in suspendierter Form und in einer Konzentration von ca. 5 % bis 20 %, vorzugsweise ca. 10 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole.

Zur parenteralen Verabreichung eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Lösungen, wie sie z.B. für die parenterale Verabreichung verwendet werden, können auch als Infusionslösungen angewandt werden.

Bevorzugte Konservierungsmittel sind z. B. Antioxidantien, wie Ascorbinsäure, oder Microbizide, wie Sorbinsäure oder Benzoesäure.

Salben sind Öl-in-Wasser-Emulsionen, die bis zu 70 %, vorzugsweise jedoch 20 - 50 % Wasser oder wässrige Phase aufweisen. Als Fettphase kommen in erster Linie Kohlenwasserstoffe, z.B. Vaseline®, Paraffinöl oder Hartparaffine in Frage, die zur Verbesserung des Wasserbindungsvermögens vorzugsweise geeignete Hydroxyverbindungen

wie Fettalkohole oder Ester davon, z.B. Cetylalkohol oder Wollwachsalkohole, wie Wollwachs, enthalten. Emulgatoren sind entsprechende lipophile Substanzen, wie Sorbitan-fettsäureester (Spans®), z.B. Sorbitanoleat und/oder Sorbitanisostearat. Zusätze zur Wasserphase sind z.B. Feuchthaltungsmittel, wie Polyalkohole, z.B. Glycerin, Propylenglykol, Sorbit und/oder Polyethylenglycol, oder wie Konservierungsmittel und Riechstoffe.

Fettsalben sind wasserfrei und enthalten als Grundlage insbesondere Kohlenwasserstoffe, z.B. Paraffin, Vaseline® oder Paraffinöl, ferner natürliche oder partialsynthetische Fette, z.B. Kokosfettsäuretriglycerid, oder vorzugsweise gehärtete Öle, z.B. hydriertes Erdnussoder Ricinusöl, ferner Fettsäurepartialester des Glycerins, z.B. Glycerinmono- und/oder -distearat, sowie z.B. die im Zusammenhang mit den Salben erwähnten, die Wasseraufnahme steigernden Fettalkohole, Emulgatoren und/oder Zusätze.

Crèmes sind Öl-in-Wasser-Emulsionen, die mehr als 50 % Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline® (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Ethylenoxyaddukte davon, wie Polyglycerinsäurefettsäureester oder Polyethylensorbitan-fettsäureester (Tween®), ferner Polyoxyethylen-fettalkoholether oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Cremes vermindern, z.B. Polyalkohole, wie Glycerin, Sorbit, Propylenglykol und/oder Polyethylenglykole, ferner Konservierungsmittel und Riechstoffe.

Pasten sind Crèmes und Salben mit sekretabsorbierenden Puderbestandteilen, wie Metalloxiden, z.B. Titanoxid oder Zinkoxid, ferner Talk und/oder Aluminiumsilikate, welche die Aufgabe haben, vorhandene Feuchtigkeit oder Sekrete zu binden.

Schäume werden aus Druckbehältern verabreicht und sind in Aerosolform vorliegende flüssige Öl-in-Wasser-Emulsionen, wobei halogenierte Kohlenwasserstoffe, wie Chlorfluorniederalkane, z.B. Dichlordifluormethan und Dichlortetrafluorethan, oder vorzugsweise nichthalogenierte gasförmige Kohlenwasserstoffe, Luft, $N_2O$ oder Kohlendioxid als Treibgase verwendet werden. Als Ölphase verwendet man u.a. die oben bei Salben und Crèmes verwendeten, ebenso die dort genannten Zusätze.

Tinkturen und Lösungen weisen meist eine wässrig-ethanolische Grundlage auf, der u.a. Polyalkohole, z.B. Glycerin, Glykole, und/oder Polyethylenglykol, als Feuchthaltemittel zur Herabsetzung der Verdunstung, und rückfettende Substanzen, wie Fettsäureester mit niederen Polyethylenglykolen, d.h. im wässrigen Gemisch lösliche, lipophile Substanzen als Ersatz für die der Haut mit dem Ethanol entzogenen Fettsubstanzen, und, falls nötig, andere Hilfs- und Zusatzmittel beigegeben sind.

Die Verbindungen der Formel I können als solche oder in Form pharmazeutischer Präparate prophylaktisch oder therapeutisch verabreicht werden, vorzugsweise in einer gegen die genannten Krankheiten wirksamen Menge an einen Warmblüter, z. B. Menschen, der einer derartigen Behandlung bedarf, wobei man sie insbesondere in Form von pharmazeutischen Präparaten verwendet. Dabei wird bei einem Körpergewicht von etwa 70 kg eine tägliche Dosis von etwa 0,1 g bis etwa 5 g, vorzugsweise von etwa 0,5 g bis etwa 2 g, einer Verbindung der vorliegenden Erfindung verabreicht.

## Beispiele

Die nachfolgenden Beispiele dienen zur Illustration der Erfindung, ohne deren Umfang einzuschränken.

Verwendete Abkürzungen:

| | |
|---|---|
| DC | Dünnschichtchromatogramm |
| FAB-MS | Fast Atom Bombardment Mass Spectroscopy |
| ges. | gesättigt |
| h | Stunde(n) |
| Hexan | n-Hexan |
| min | Minute(n) |
| RT | Raumtemperatur |
| Smp. | Schmelzpunkt |
| THF | Tetrahydrofuran |

Soweit nicht anders angegeben, werden Gemische von Flüssigkeiten durch ihre Volumenanteile gekennzeichnet (v/v).

Bei $^1$H-NMR Spektren werden chemische Verschiebungen in ppm als $\delta$-Wert gekennzeichnet.

Die nachfolgend genannten Vorstufen betreffen zur Synthese der Beispiele benötigte Ausgangsverbindungen.

Vorstufe 1-1:

4-Hydroxy-5,6-dimethyl-7-benzyl-pyrrolo[2,3-d]pyrimidin

9,5 g 2-Amino-4,5-dimethyl-1-benzyl-3-cyano-pyrrol (hergestellt aus Acetoin, Benzylamin und Malonodinitril nach einem bekannten Verfahren (siehe H.J Roth und K. Eger, Arch. Pharmaz. 308, 179 (1975))) werden mit 80 ml 85%-iger Ameisensäure 5 Stunden bei 110°C gekocht. Die Reaktionslösung wird im Eisbad abgekühlt, wobei hellbraune Kristalle ausfallen. Die Suspension wird auf ca. 200 ml Eiswasser gegossen und ca. 10 min gerührt. Dann wird der Niederschlag abgenutscht. Die Kristalle werden mit Wasser und anschliessend mit Hexan gewaschen und getrocknet. Man erhält die Titelverbindung vom Smp. 251-253 °C (Zersetzung).

Vorstufe 2-1:

4-Hydroxy-5,6-tetramethylen-7-benzyl-pyrrolo[2,3-d]pyrimidin

In analoger Weise wie für Vorstufe 1-1 beschrieben wird aus 15 g 2-Amino-1-benzyl-3-cyano-4,5,6,7-tetrahydroindol und 100 ml 85 %-iger Ameisensäure 4-Hydroxy-5,6-tetramethylen-7-benzyl-pyrrolo[2,3-d]pyrimidin als weisse Kristalle vom Smp. 104-105 °C erhalten.
$C_{17}H_{17}N_3O$: FAB-MS (M+H)+ = 280

Analog wird hergestellt:

Vorstufe 3-1: 4-Hydroxy-5,6-diphenyl-7-benzyl-pyrrolo[2,3-d]pyrimidin

Herstellung aus 2-Amino-4,5-diphenyl-1-benzyl-3-cyano-pyrrol (hergestellt aus Benzoin, Benzylamin und Malodinitril analog wie in Vorstufe 1-1) durch Kochen in 85%-iger Ameisensäure analog Vorstufe 1-1.

Smp.: 225-230 °C
$C_{25}H_{19}N_3O$: FAB-MS (M+H)+ = 378

Vorstufe 1-2: 4-Chlor-5,6-dimethyl-7-benzyl-pyrrolo[2,3-d]pyrimidin

2,5 g 4-Hydroxy-5,6-dimethyl-7-benzyl-pyrrolo[2,3-d]pyrimidin werden mit 20 ml $POCl_3$ während 2,5 Stunden am Rückfluss gekocht. Die braune Lösung wird auf RT abgekühlt und auf Eiswasser gegossen. Der bräunliche Niederschlag wird abgenutscht und in Essigsäureethylester gelöst. Die Essigsäureethylesterphase wirde mit Wasser gewaschen, getrocknet und am Rotationsverdampfer konzentriert, bis weisse Kristalle ausfallen. Es wird 4-Chlor-5,6-dimethyl-7-benzyl-pyrrolo[2,3-d]pyrimidin als weisse Kristalle vom Smp. 115-116 °C erhalten.
$C_{15}H_{14}N_3Cl$: FAB-MS (M+H)+ = 272

Vorstufe 2-2: 4-Chlor-5,6-tetramethylen-7-benzyl-pyrrolo[2,3-d]pyrimidin

In analoger Weise wie für Vorstufe 1-2 beschrieben werden aus 8.3 g 4-Hydroxy-5,6-tetramethylen-7-benzyl-pyrrolo[2,3-d]pyrimidin und 50 ml $POCl_3$ rohe Kristalle des Produktes erhalten. Nach Umkristallisation aus Ethanol wird 4-Chlor-5,6-tetramethylen-7-benzyl-pyrrolo[2,3-d]pyrimidin in Form von hellrosa Kristallen vom Smp. 104-105 °C erhalten.
$C_{17}H_{16}N_3Cl$: FAB-MS (M+H)+ = 298

Analog wird aus Vorstufe 3-1 hergestellt:

Vorstufe 3-2: 4-Chlor-5,6-diphenyl-7-benzyl-pyrrolo[2,3-d]pyrimidin

Smp.: 181-183 °C
$C_{25}H_{18}ClN_3$: FAB-MS (M+H)+ = 396.

Vorstufe 1-3: 4-(m-Chloranilino)-5,6-dimethyl-7-benzyl-pyrrolo[2,3-d]pyrimidin

0,6 g 4-Chlor-5,6-dimethyl-7-benzyl-pyrrolo[2,3-d]pyrimidin und 0,28 ml m-Chloranilin werden in 10 ml Ethanol 17 Stunden am Rückfluss erhitzt. Die braune Lösung wird zur Trockene eingedampft, der Rückstand in Essigsäureethylester aufgenommen, die Essigsäureethylesterlösung mit Natriumbicarbonatlösung und Wasser neutral gewaschen,

getrocknet und eingedampft. Der Rückstand wird aus Essigsäureethylester/Hexan kristallisiert. Es wird 4-(m-Chloranilino)-5,6-dimethyl-7-benzyl-pyrrolo[2,3-d]pyrimidin als weisse Kristalle vom Smp. 132-133 °C erhalten.

$C_{21}H_{19}N_4Cl$: FAB-MS (M+H)+ = 363

Ausgehend von 4-Chlor-5,6-dimethyl-7-benzyl-pyrrolo[2,3-d]pyrimidin werden analog Vorstufe 1-3 hergestellt:

Vorstufe 1-4: 4-(m-Bromanilino)-5,6-dimethyl-7-benzyl-pyrrolo[2,3-d]pyrimidin

Smp.: 131-135 °C

$C_{21}H_{19}BrN_4$: FAB-MS (M+H)+ = 407

Vorstufe 1-5: 4-(m-Fluoranilino)-5,6-dimethyl-7-benzyl-pyrrolo[2,3-dipyrimidin

Smp.: 118-120°C

$C_{21}H_{19}FN_4$: FAB-MS (M+H)+ = 347

Vorstufe 1-6: 4-(m,m-Dichloranilino)-5,6-dimethyl-7-benzyl-pyrrolo[2,3-d]pyrimidin

Smp.: 188-189 °C

$C_{20}H_{18}Cl_2N_4$: FAB-MS (M+H)+ = 386

**Beispiel 1: 4-(m-Chloranilino)-5,6-dimethyl-7H-pyrrolo[2,3-d]pyrimidin**

1 g (2,76 mMol) 4-(m-Chloranilino)-5,6-dimethyl-7-benzyl-pyrrolo[2,3-d]pyrimidin und 2,57 g (19.32 mMol) $AlCl_3$ werden in 20 ml Toluol während 2 Stunden am Rückfluss erhitzt, bis im DC alles Ausgangsmaterial verschwunden ist. Die Reaktionslösung wird auf RT abgekühlt, auf Eiswasser gegossen und 2 Stunden bei 0 °C gerührt. Der Niederschlag wird abgenutscht und in heissem Essigsäureethylester gelöst. Die Essigsäureethylesterlösung wird je zweimal mit 5% Natriumbicarbonatlösung und ges. NaCl-Lösung gewaschen, getrocknet und eingedampft. Der Rückstand wird aus Essigsäureethylester/Hexan kristallisiert, wobei 4-(m-Chloranilino)-5,6-dimethyl-7H-pyrrolo[2,3-d]pyrimidin als farblose Kristalle vom Smp. 239-240°C erhalten wird.

$C_{14}H_{13}ClN_4$: FAB-MS (M+H)+ = 273

Zur Herstellung des Hydrochlorids werden 200 mg 4-(m-Chloranilino)-5,6-dimethyl-7H-pyrrolo[2,3-d]pyrimidin in 15 ml THF gelöst und bei 0 °C tropfenweise mit 0,5 ml einer 3 N etherischen HCl-Lösung versetzt. Nach Zugabe von Ether fallen weisse Kristalle des Hydrochlorids aus. Die Kristalle werden abgenutscht, mit wenig Ether gewaschen und getrocknet.

Analog Beispiel 1 werden aus den entsprechenden Benzyl-Verbindungen folgende Verbindungen hergestellt:

**Beispiel 2: 4-(m-Bromanilino)-5,6-dimethyl-7H-pyrrolo[2,3-d]pyrimidin**

Herstellung aus Vorstufe 1-4; Ergebnis: Titelverbindung

Smp.: 243-244 °C;

$C_{14}H_{13}BrN_4$: FAB-MS (M+H)+ = 317.

**Beispiel 3:**

**4-(m-Fluoranilino)-5,6-dimethyl-7H-pyrrolo[2,3-d]pyrimidin**

Herstellung aus Vorstufe 1-5; Ergebnis: Titelverbindung

Smp.: 245-255 °C;

$C_{14}H_{13}FN_4$: FAB-MS (M+H)+ = 257.

**Beispiel 4: 4-(m,m-Dichloranilino)-5,6-dimethyl-7H-pyrrolo[2,3-d]pyrimidin**

Herstellung aus Vorstufe 1-6; Ergebnis: Titelverbindung

Smp.: >250°C;

$C_{14}H_{12}Cl_2N_4$: FAB-MS (M+H)+ = 307.

Vorstufe 1-7: 4-Chlor-5,6-dimethyl-7H-pyrrolo[2,3-d]pyrimidin

Smp.: 252-253 °C
$C_8H_8ClN_3$: FAB-MS (M+H)+ = 182

Vorstufe 2-3: 4-Chlor-5,6-tetramethylen-7H-pyrrolo[2,3-d]pyrimidin

Smp.: 244 - 245 °C
$C_{10}H_{10}ClN_3$: FAB-MS (M+H)+ = 182

Vorstufe 3-3: 4-Chlor-5,6-diphenyl-7H-pyrrolo[2,3-d]pyrimidin

Smp.: 228 - 230 °C
$C_{18}H_{12}ClN_3$: FAB-MS (M+H)+ = 306

Analog der Reaktion zur Herstellung von Vorstufe 1-3 werden ausgehend von 4-Chlor-5,6-dimethyl-7H-pyrrolo [2,3-d]pyrimidin(Vorstufe 1-7) und dem entsprechenden meta-substituierten Anilin durch Kochen in n-Butanol die folgenden Verbindungen hergestellt:

**Beispiel 5: 4-(m-Methylanilino)-5,6-dimethyl-7H-pyrrolo[2,3-d]pyrimidin**

Smp.: 230-234 °C;
$C_{15}H_{16}N_4$: FAB-MS (M+H)+ = 253.

**Beispiel 6: 4-(m-Methoxyanilino)-5,6-dimethyl-7H-pyrrolo[2,3-d]pyrimidin**

Smp.: 209-214 °C
$C_{15}H_{15}N_4O$: FAB-MS: (M+H)+ = 269

In analoger Weise wie in Vorstufe 1-3 beschrieben werden ausgehend von 4-Chlor-5,6-tetramethylen-7-benzyl-pyrrolo[2,3-d]pyrimidin(Vorstufe 2-2) durch Reaktion mit dem entsprechenden meta-substituierten Anilin die folgenden Verbindungen hergestellt:

Vorstufe 2-4: 4-(m-Chloranilino)-5,6-tetramethylen-7-benzyl-pyrrolo[2,3-d]pyrimidin

Smp.: 145-147 °C
$C_{23}H_{21}ClN_4$: FAB-MS: (M+H)+ = 389

Vorstufe 2-5: 4-(m-Bromanilino)-5,6-tetramethylen-7-benzyl-pyrrolo[2,3-d]pyrimidin

Smp.: 159-161 °C
$C_{23}H_{21}BrN_4$: FAB-MS: (M+H)+ = 434

Vorstufe 2-6: 4-(m-Fluoranilino)-5,6-tetramethylen-7-benzyl-pyrrolo[2,3-d]pyrimidin

Smp.: 131-132 °C
$C_{23}H_{21}FN_4$: FAB-MS: (M+H)+ = 373

Die Abspaltung der Benzyl-Schutzgruppe erfolgt analog Beispiel 1. Es werden erhalten:

**Beispiel 7: 4-(m-Chloranilino)-5,6-tetramethylen-7H-pyrrolo[2,3-d]pyrimidin**

Smp.: 246-249 °C
$C_{16}H_{15}ClN_4$: FAB-MS: (M+H)+ = 299

**Beispiel 8: 4-(m-Bromanilino)-5,6-tetramethylen-7H-pyrrolo[2,3-d]pyrimidin**

Smp.: 240-245 °C
$C_{16}H_{15}BrN_4$: FAB-MS: (M+H)+ = 343

**Beispiel 9: 4-(m-Fluoranilino)-5,6-tetramethylen-7H-pyrrolo[2,3-d]pyrimidin**

Smp.: >240 °C C

$C_{16}H_{15}FN_4$: FAB-MS: (M+H)+ = 283

Analog wie für Vorstufe 1-3 beschrieben werden ausgehend von 4-Chlor-5,6-tetramethylen-7H-pyrrolo[2,3-d]pyrimidin (Vorstufe 2-3) und dem entsprechenden meta-substituierten Anilin durch Kochen in n-Butanol die folgenden Verbindungen hergestellt:

**Beispiel 10: 4-(m-Methylanilino)-5,6-tetramethylen-7H-pyrrolo[2,3-d]pyrimidin**

Smp.: 258 - 261 °C

$C_{17}H_{18}N_4$: FAB-MS: (M+H)+ = 279

**Beispiel 11: 4-(m-Methoxyanilino)-5,6-tetramethylen-7H-pyrrolo[2,3-d]pyrimidin**

Smp.: 239-241 °C

$C_{17}H_{18}N_4O$: FAB-MS: (M+H)+ = 295

Analog werden aus 4-Chlor-5,6-diphenyl-7H-pyrrolo[2,3-d]pyrimidin (Vorstufe 3-3) die folgenden Verbindungen hergestellt:

**Beispiel 12: 4-(m-Chloranilino)-5,6-diphenyl-7H-pyrrolo[2,3-d]pyrimidin**

Smp.: 270 °C

$C_{24}H_{17}ClN_4$: FAB-MS: (M+H)+ = 396

**Beispiel 13: 4-(m-Bromanilino)-5,6-diphenyl-7H-pyrrolo[2,3-d]pyrimidin**

Smp.: > 260 °C

$C_{24}H_{17}BrN_4$: FAB-MS: (M+H)+ = 441

**Beispiel 14: 4-(m-Trifluormethyanilino)-5,6-tetramethylen-7H-pyrrolo[2,3-d]pyrimidin**

150 mg (0.85 mMol) 4-Hydroxy-5,6-tetramethylen-7-phenethyl-pyrrolo[2,3-d]pyrimidin (hergestellt durch Ringschluss von 2-Amino-1-phenethyl-3-cyano-4,5,6,7-tetrahydroindol mit 85 %-iger Ameisensäure analog Vorstufe 1-1) werden analog zu einem bekannten Verfahren (siehe J. Heterocycl. Chem. 22, 859 (1985)) mit 483 mg (3.4 mMol) Phosphorpentoxid, 468 mg (3.4 mMol)) Triethylamin-hydrochlorid und 675 mg (3.4mMol) 3-Trifluormethylanilin ca. 5 Stunden auf 240 °C erhitzt. Dann werden bei 80-100 °C unter Rühren 15 ml 2N NaOH zugegeben. Der klebrige Niederschlag wird abfiltriert und die Mutterlauge mehrmals mit Essigsäureethylester extrahiert. Die Essigsäureethylesterphase wird mit Wasser gewaschen, getrocknet und eingedampft. Der Niederschlag und der Essigsäureethylesterrückstand werden zusammen über 60 g Silicagel chromatographiert. Elution mit Methylenchlorid-Methanol (95:5) ergibt 4-(m-Trifluormethylanilino)-5,6-tetramethylen-7H-pyrrolo[2,3-d]pyrimidin als hellgelbe Kristalle vom Smp. 259-261 °C. $C_{17}H_{15}F_3N_4$: FAB-MS (M+H)+ = 333.

Analog zu Beispiel 14 wird hergestellt:

**Beispiel 15: 4-(m,p-Dichloranilino)-5,6-tetramethylen-7H-pyrrolo[2,3-d]pyrimidin**

Herstellung unter Verwendung von m,p-Dichloranilin anstelle von 3-Trifluormethylanilin; Ergebnis: Titelverbindung

Smp.: > 270 °C

$C_{16}H_{14}Cl_2N_4$: FAB-MS: (M+H)+ = 333

Die nachfolgenden Beispiele **16 bis 22** beziehen sich auf die Verwendung bereits bekannter Verbindungen der Formel I (siehe Jorgensen, A., et al., J. Heterocyclic Chem. 22, 859 (1985)):

Die Testsysteme sind wie folgt identifiziert:

Test A) Hemmwirkung auf EGF-R ICD (ICD = intrazelluläre Domäne): Das Testsystem ist oben beschrieben. Das Ergebniss wird angegeben als $IC_{50}$ in μM (Konzentration an Wirkstoff bei halbmaximaler Hemmung).

Test B) Hemmwirkung auf das Wachstum von MK-Zellen: Das Testsystem ist oben im Detail beschrieben. Das Ergebnis wird angegeben als $IC_{50}$ in μM (Konzentration an Wirkstoff bei halbmaximaler Hemmung).

Test C) Hemmwirkung auf das A431-Tumorwachtum *in vivo:* Das Testsystem ist oben im Detail beschrieben. Das Ergebnis (als T/C %) und nähere Versuchsbedingungen werden tabellarisch angegeben.

**Beispiel 16: 4-Anilino-5,6-dimethyl-7H-pyrrolo[2,3-d]pyrimidin**

Test A): 2 µM
Test B): 24,6 µM

**Beispiel 17: 4-(p-Methylanilino)-5,6-dimethyl-7H-pyrrolo[2,3-d]pyrimidin**

Test A): 1,9 µM
Test B): 24,6 µM

**Beispiel 18: 4-(m,p-Dichloranilino)-5,6-dimethyl-7H-pyrrolo[2,3-d]pyrimidin**

Test A): 0,070 µM
Test B): 34,8 µM

**Beispiel 19: 4-(o-Fluoranilino)-5,6-dimethyl-7H-pyrrolo[2,3-d]pyrimidin**

Test A): 1,6 µM
Test B): 49,3 µM

**Beispiel 20: 4-(p-Fluoranilino)-5,6-dimethyl-7H-pyrrolo[2,3-d]pyrimidin**

Test A): 0,56 µM
Test B): 13,1 µM

**Beispiel 21: 4-Anilino-5,6-tetramethylen-7H-pyrrolo[2,3-d]pyrimidin**

Test A): 0,31 µM
Test B): 12,8 µM

**Beispiel 22: 4-(4-Methylanilino)-5,6-tetramethylen-7H-pyrrolo[2,3-d]pyrimidin**

Test A): 0,11 µM
Test B): 13,9 µM

**Beispiel 23: Trockenkapseln**

5000 Kapseln werden hergestellt, welche jeweils als Wirkstoff 0,25 g einer der in den Beispielen 1 bis 22 oder 26 bis 31 genannten Verbindungen der Formel I enthalten:

| Zusammensetzung | |
|---|---|
| Wirkstoff | 1250 g |
| Talkum | 180 g |
| Weizenstärke | 120 g |
| Magnesiumstearat | 80 g |
| Lactose | 20g |

Herstellungsverfahren: Die pulverisierten genannten Substanzen werden durch ein Sieb mit einer Maschenweite von 0,6 mm gepresst. Portionen von 0,33 g der Mischung werden in Gelatinekapseln mit Hilfe einer Kapsel-Füllmaschine abgefüllt.

**Beispiel 24: Weichkapseln**

5000 Weichgelatine-Kapseln, die jeweils 0,05 g eine der in den Beispielen 1 bis 22 oder 26 bis 31 genannten Verbindungen der Formel I als Wirkstoff enthalten, werden hergestellt:

| Zusammensetzung | |
|---|---|
| Wirkstoff | 250 g |
| Lauroglykol | 2l |

Herstellungsverfahren: Der pulverisierte Wirkstoff wird in ®Lauroglykol (Propylenglykol-Laurat, Gattefossé S.A., Saint Priest, Frankreich) suspendiert und in einem Nass-Pulverisator zu einer Korngrösse von etwa 1 bis 3 µm gemahlen. Portionen von jeweils 0,419 g der Mischung werden dann in Weichgelatinekapseln mittels einer Kapsel-Füllmaschine abgefüllt.

**Beispiel 25: Weichkapseln**

5000 Weichgelatine-Kapseln, die jeweils 0,05 g eine der in den Beispielen 1 bis 22 oder 26 bis 31 genannten Verbindungen der Formel I als Wirkstoff enthalten, werden hergestellt:

| Zusammensetzung | |
|---|---|
| Wirkstoff | 250 g |
| PEG 400 | 1 l |
| Tween 80 | 1 l |

Herstellungsverfahren: Der pulverisierte Wirkstoff wird in PEG 400 (Polyethylenglykol mit $M_r$ zwischen etwa 380 und etwa 420, Fluka, Schweiz) und ®Tween 80 (Polyoxyethylen-Sorbitan-Monolaurat, Atlas Chem. Ind., Inc., USA, geliefert von Fluka, Schweiz) suspendiert und in einem Nass-Pulverisator zu einer Korngrösse von etwa 1 bis 3 µm gemahlen. Portionen von jeweils 0,43 g der Mischung werden dann in Weichgelatinekapseln mittels einer Kapsel-Füllmaschine abgefüllt.

Vorstufe 4-1: 2-Amino-3-carboxyethyl-5-(4-methoxyphenyl)-pyrrol

In einem trockenen Dreihalskolben werden unter Argon 10 ml abs. Ethanol und 1,668 g (10 mmol) Amidinoessigsäureethylester·HCl vorgelegt, die Mischung auf 0 - 5 $^0$C abgekühlt und mit 716 mg (10 mMol) Nartiumethylat (95 %) versetzt. Anschliessend gibt man 1,145 g (5 mMol) 4-Methoxyphenacylbromid (Fluka, Buchs, Schweiz) hinzu und lässt auf RT erwärmen. Dann wird für 50 h weitergerührt. Das Reaktionsgemisch wird anschliessend in einer Emulsion aus Wasser und Essigsäureethylester aufgenommen. Man extrahiert die organische Phase noch 3 mal mit Wasser, dann einmal mit gesättigter NaCl-Lösung. Die wässrigen Phasen werden vereint und mit Essigsäureethylester rückextrahiert. Die vereinigten organischen Phasen werden über Magnesiumsulfat getrocknet, filtriert und zur Trockene eingedampft. Der resultierende Rückstand wird mittels "Flash-Chromatographie" auf einer Kieselgel-60-Säule (40 mg; Merck, Darmstadt, Bundesrepublik Deutschland) gereinigt. Als Laufmittel wird hierbei Essigsäureethylester:Hexan (1:1) verwendet. Die Produktfraktionen werden vereinigt, zur Trockne eingedampft und mit Diethylether/n-Hexan verrührt. Das Produkt wird abgesaugt und mit n-Hexan gewaschen. Nach Trocknung am Hochvakuum erhält man die Titelverbindung, Smp. 141-142 °C; FAB-MS: $(M+H)^+ = 260$.

(Die Herstellung von Amidinoessigsäureethylester·HCl erfolgt aus Cyanessigsäureethylester (Fluka, Buchs, Schweiz) durch Umsetzung in HCl und Ethanol, Rühren der Suspension für 22 h, Zugabe von Ether, erneutes Rühren für 10 min, kaltes Abfiltrieren der erhaltenen Kristalle des erhaltenen 3-Ethoxy-3-iminopropansäureethylesters; Zugabe dieser Kristalle zu ammoniakgesättigtem Ethanol und Rühren der Suspension, Filtrieren der Suspension und Zugabe von Aceton und erneutes Filtrieren; und Zugabe von HCl in Diethylether zu den Filtraten, wobei das Amidinoessigsäureethylester·HCl-Salz ausfällt, das dann weiterverwendet wird).

Analog Vorstufe 4-1 werden folgende Ausgangsverbindungen hergestellt:

Vorstufe 5-1: 2-Amino-3-carboxvethyl-(2,5-dimethoxyphenyl)pyrrol

(Ausgangsmaterialien: Amidinoessigsäureethylester·HCl und 2-Bromo-2',5'-dimethoxy-acetophenon [Aldrich, Buchs, Schweiz])

Titelverbindung: Smp.: 110-111 °C; FAB-MS: $(M+H)^+$= 291.

Vorstufe 6-1: 2-Amino-3-carboxyethyl-5-(phenyl)-pyrrol

(Ausgangsmaterialien: Amidinoessigsäureethylester·HCl und Phenacylbromid (Aldrich, Buchs, Schweiz))
Titelverbindung: FAB-MS: $(M+H)^+$= 231; $^1$H-NMR (DMSO): δ=7,5 (d, 2H); 7,3 (t, 2H); 7,1 (m, 1H); 6,5 (s, 1H), 5,7 (s, 2H), 4,15 (m, 2H); 1,25 (m, 3H).

Vorstufe 7-1: 2-Amino-3-carboxyethyl-4-methyl-5-(4-methoxyphenyl)-pyrrol

(Ausgangsmaterialien: Amidinoessigsäureethylester·HCl und 2-Bromo-4'-methoxypropiophenon [hergestellt aus 4'-Methoxypropiophenon {Aldrich, Buchs, Schweiz} durch Bromierung mit $Br_2/CH_3COOH$, siehe Chem. Ber. 22, 3251 (1889)]
Titelverbindung: FAB-MS: $(M+H)^+$= 275

Vorstufe 8-1: 2-Amino-3-carboxyethyl-5-(3-methoxvphenyl)-pyrrol

(Ausgangsmaterialien: Amidinoessigsäureethylester·HCl und 3-Methoxyphenacylbromid (Aldrich, Buchs, Schweiz))
Titelverbindung: Smp.: 96-97 °C; $^1$H-NMR (DMSO): δ= 7,2 (m, 1H); 7,05 (m, 2H); 6,65 (m, 1H); 6,5 (m, 1H); 5,7 ($NH_2$); 4,13 (q, 2H); 3,86 (s, 3H); 1,23 (t, 3H).

Vorstufe 9-1: 2-Amino-3-carboxyethyl-5-(2-methoxyphenyl)-pyrrol

(Ausgangsmaterialien: Amidinoessigsäureethylester·HCl und 2-Methoxyphenacylbromid (Aldrich, Buchs, Schweiz))
Titelverbindung: Smp.: 128 °C; $^1$H-NMR (DMSO): δ= 10,3 (1H); 7,45 (m, 1H); 6,8-7,2 (m, 3H); 6,53 (m, 1H); 5,7 ($NH_2$); 4,15 (q, 2H); 3,86 (s, 3H); 1,25 (t, 3H).

Vorstufe 4-2: 4-Hydroxy-6-(4-methoxyphenyl)-*7H*-pyrrolo[2,3-d]pyrimidin

610,7 mg (2,5 mMol) 2-Amino-3-carboxyethyl-5-(4-methoxyphenyl)pyrrol (Vorstufe 4-1), 5 ml Formamid, 2,5 ml N,N-Dimethylformamid und 1,25 ml Ameisensäure werden zusammen für 16 Stunden bei 150 °C gerührt. Zur warmen Reaktionsmischung wird ein wenig Isopropanol gegeben. Nach dem Ankühlen der Reaktionsmischung wird das ausgefallene Produkt abfiltriert. Es wird mit wenig Isopropanol und 2 mal mit je 10 ml Hexan gewaschen. Nach Trocknen am Hochvakuum erhält man die Titelverbindung als hell-beige Kristalle; Smp. >300 °C, $^1$H-NMR (DMSO): δ= 7,87 (1H); 7,80 (1H); 7,16 (1H); 7,02 - 6,95 (2H); 6,8 (1H); 3,8 (3H); FAB-MS: $(M+H)^+$= 242.

Analog werden aus den Titelverbindungen der Vorstufen 5-1 bis 9-1 die nachfolgenden Titelverbindungen der Vorstufen 5-2 bis 9-2 hergestellt:

Vorstufe 5-2: 4-Hydroxy-6-(2,5-dimethoxyphenyl)-*7H*-pyrrolo[2,3-d]pyrimidin

Aus Vorstufe 5-1 wird die Titelverbindung erhalten:
Smp.: > 300 °C; FAB-MS: $(MH)^+$=272.

Vorstufe 6-2: 4-Hydroxy-6-(phenyl)-*7H*-pyrrolo[2,3-d]pyrimidin

Aus Vorstufe 6-1 wird die Titelverbindung erhalten:
Smp.: > 300 °C; FAB-MS: $(M+H)^+$=2=2

Vorstufe 7-2: 4-Hydroxy-5-methyl-6-(4-methoxyphenyl)-*7H*-pyrrolo[2,3-d]pyrimidin

Aus Vorstufe 7-1 wird die Titelverbindung erhalten:
FAB-MS: $(M+H)^+$=256

Vorstufe 8-2: 4-Hydroxy-6-(3-methoxyphenyl)-*7H*-pyrrolo[2,3-d]pyrimidin

Aus Vorstufe 8-1 wird die Titelverbindung erhalten:

Smp.: > 300 °C; $^1$H-NMR (DMSO): δ=7,9 (1H); 7,28-7,46 (m, 3H); 6,98 (s, 1H); 6,85 (m, 1H); 3,83 (s, 3H).

Vorstufe 9-2: 4-Hydroxy-6-(2-methoxyphenyl)-*7H*-pyrrolo[2,3-d]pyrimidin

Aus Vorstufe 9-1 wird die Titelverbindung erhalten:
Smp.: > 300 °C; $^1$H-NMR (DMSO): δ= 12 (1H); 7,9 (1H), 7,77 (m, 1H); 6,95 - 7,4 (m, 4H); 3,95 (3H).

Die Titelverbindungen der Vorstufen 4-2 bis 9-2 werden dann analog Vorstufe 1-2 mit $POCl_3$ zu den folgenden Vorstufen 4-3 bis 9-3 umgesetzt:

Vorstufe 4-3: 4-Chlor-6-(4-methoxyphenyl)-*7H*-pyrrolo[2,3-d]pyrimidin

Aus Vorstufe 4-2 wird die Titelverbindung erhalten:
Smp.: 248-249 °C; FAB-MS: $(M+H)^+$=260.

Vorstufe 5-3: 4-Chlor-6-(2,5-4-Chlor-6-(2,5-dimethoxyphenyl)-*7H*-pyrrolo[2,3-d]pyrimidin

Aus Vorstufe 5-2 wird die Titelverbindung erhalten:
$^1$H-NMR (DMSO): δ= 8,6 (s, 1H); 7,5 (d, 1H); 7,15 (d, 1H); 7,1 (s, 1H); 7,0 (m, 1H); 3,9 (s, 3H); 3,8 (s, 3H).

Vorstufe 6-3: 4-Chlor-6-(phenyl)-*7H*-pyrrolo[2,3-d]pyrimidin

Aus Vorstufe 6-2 wird die Titelverbindung erhalten:
FAB-MS: $(M+H)^+$= 230; $^1$H-NMR (DMSO): δ= 8,6 (s, 1H); 8,05 (d, 2H); 7,5 (m, 3H); 7,1 (s, 1H).

Vorstufe 7-3: 4-Chlor-5-methyl-6-(4-methoxyphenyl)-*7H*-pyrrolo[2,3-d]pyrimidin

Aus Vorstufe 7-2 wird die Titelverbindung erhalten:
FAB-MS: $(M+H)^+$= 274.

Vorstufe 8-3: 4-Chlor-6-(3-methoxyphenyl)-*7H*-pyrrolo[2,3-d]pyrimidin

Aus Vorstufe 8-2 wird die Titelverbindung erhalten:
(wird nicht isoliert, sondern als Rohprodukt weiterverwendet)

Vorstufe 9-3: 4-Chlor-6-4-Chlor-6-(2-methoxyphenyl)-*7H*-pyrrolo[2,3-d]pyrimidin

Aus Vorstufe 9-2 wird die Titelverbindung erhalten:
(wird nicht isoliert, sondern als Rohprodukt weiterverwendet)

Analog der Vorstufe 1-3 wird die jeweilige Titelverbindung aus Vorstufe 5-3, 6-3, 7-3, 8-3 oder 9-3 mit m-Chloranilin zu den folgenden Beispielen (= 4-(m-Chloranilino)-7*H*-pyrrolo[2,3-d]pyrimidinen) umgesetzt:

**Beispiel 26: 4-(m-Chloranilino)-6-(4-methoxyphenyl)-*7H*-pyrrolo[2,3-d]pyrimidin**

Aus Vorstufe 4-3 wird die Titelverbindung erhalten:
Smp.: 294-295 °C; FAB-MS: $(M+H)^+$= 351.

**Beispiel 27: 4-(m-Chloranilino)-6-(2,5-dimethoxyphenyl)-*7H*-pyrrolo[2,3-d]pyrimidin**

Aus Vorstufe 5-3 wird die Titelverbindung erhalten:
Smp.: 267-268 °C; FAB-MS: $(M+H)^+$= 381.

**Beispiel 28: 4-(m-Chloranilino)-6-(phenyl)-*7H*-pyrrolo[2,3-d]pyrimidin**

Aus Vorstufe 6-3 wird die Titelverbindung erhalten:
Smp.: 285-286 °C; FAB-MS: $(M+H)^+$= 321.

**Beispiel 29: 4-(m-Chloranilino)-5-methyl-6-(4-methoxyphenyl)-*7H*-pyrrolo[2,3-d]pyrimidin**

Aus Vorstufe 7-3 wird die Titelverbindung erhalten:
FAB-MS: $(M+H)^+$= 365.

**Beispiel 30: 4-(m-Chloranilino)-6-(3-methoxyphenyl)-*7H*-pyrrolo[2,3-d]pyrimidin**

Aus Vorstufe 8-3 wird die Titelverbindung erhalten:
Smp.: 262-263 °C; FAB-MS: $(M+H)^+$= 351.

**Beispiel 31: 4-(m-Chloranilino)-6-(2-methoxyphenyl)-*7H*-pyrrolo[2,3-d]pyrimidin**

Aus Vorstufe 9-3 wird die Titelverbindung erhalten:
Smp.: 221-222 °C; FAB-MS: $(M+H)^+$= 351.

**Beispiel 32: Hemmwirkung auf die intrazelluläre Domäne des EGF-R (ICD)**

Das Testsystem ist oben beschrieben. Das Ergebniss wird angegeben als $IC_{50}$ in µM (Konzentration an Wirkstoff bei halbmaximaler Hemmung).

| Verbindung aus Beispiel | $IC_{50}$ |
|---|---|
| 1 | 0,045 |
| 2 | 0,025 |
| 3 | 0,55 |
| 4 | 0,17 |
| 5 | 0,57 |
| 6 | 1,2 |
| 7 | 0,033 |
| 8 | 0,046 |
| 9 | 0,2 |
| 10 | 0,82 |
| 11 | 0,35 |
| 12 | 0,096 |
| 13 | 0,033 |
| 14 | 0,36 |
| 15 | 0,15 |
| 26 | 0,015 |
| 27 | 0,033 |
| 28 | 0,013 |
| 29 | 0,43 |

| | |
|---|---|
| 30 | 1,77 |
| 31 | 0,019 |

**Beispiel 33: Hemmwirkung auf das A431-Tumorwachstum in vivo:**

Das Testsystem ist oben im Detail beschrieben. Das Ergebnis (als T/C %) und nähere Versuchsbedingungen werden tabellarisch angegeben:

Tiere Weibliche Balb/c nu/nu-Nacktmäuse (Tif/Si oder Bomholtgaard)
Formulierung: Dimethylsulfoxid/®Tween 80/0,9 % NaCl.

| Verbindung aus Bsp. | Applikation | Dosis (mg/kg) | T/C % | | |
|---|---|---|---|---|---|
| | | | Exp. 1 | Exp. 2 | Exp. 3 |
| 1 | p.o. | 125 | - | 2 | - |
| | p.o. | 50 | 14 | 9 | 14 |
| | p.o. | 25 | 16 | 14 | 16 |
| | p.o. | 12,5 | - | 16 | 21 |
| | p.o. | 6,25 | - | 23 | 32 |
| | p.o. | 3,13 | - | 29 | 41 |
| | p.o. | 1,56 | | 45 | 61 |
| | i.p. | 31,25 | - | 3 | - |
| | i.p. | 25,00 | - | - | 12 |
| | i.p. | 12,5 | 11 | 6 | 16 |
| | i.p. | 6,25 | 11 | 8 | 28 |
| | i.p. | 3,13 | - | 12 | 22 |
| | i.p. | 1,56 | - | 21 | 29 |
| | i.p. | 0,78 | - | 50 | 58 |
| | i.p. | 0,39 | - | 58 | - |

Die Behandlung beginnt am Tag 5(Exp. 1), 7 (Exp. 2) oder 6 (Exp. 3) nach der Tumortransplantation und erfolgt dann jeweils für 15 Tage einmal täglich.

| Verbindung | Applikation | Dosis (mg/kg) | T/C% |
|---|---|---|---|
| keine (Kontrolle) | - | - | 100 |
| aus Beispiel 7 | täglich ab Tag 6 bis Tag 20 | | |
| | p.o. | 50.00 | 17 |
| | p.o. | 25.00 | 28 |
| | p.o. | 12,50 | 45 |
| | p.o. | 6,25 | 55 |
| | p.o. | 3,13 | 68 |
| | p.o. | 1,56 | 84 |
| | i.p. | 50,00 | 12 |
| | i.p. | 25,00 | 15 |
| | i.p. | 12,50 | 18 |
| | i.p. | 6,25 | 41 |

(fortgesetzt)

| Verbindung | Applikation | Dosis (mg/kg) | T/C% |
|---|---|---|---|
| | i.p. | 3,13 | 61 |
| | i.p. | 1,56 | 88 |

Anmerkung: Nach i.p. Applikation (60 - 6,25 mg/kg) wird ein Teil der Verbindung nicht resorbiert.

| Verbindung aus Bsp. | Applikation | Dosis (mg/kg) | T/C % | |
|---|---|---|---|---|
| | | | Exp. 1 | Exp. 2 |
| 26 | täglich ab Tag 6 bis Tag 20 | | | |
| | i.p. | 50 | 15 | 15 |
| | i.p. | 12,5 | 32 | 57 |
| | i.p. | 3,13 | 35 | 84 |

**Patentansprüche**

**1.** Verwendung von Verbindungen der Formel I

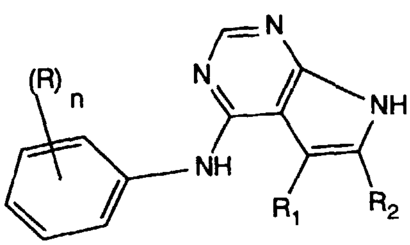

(I)

worin n = 0 bis 5 ist und R für einen Substituenten ausgewählt aus Halogen, Niederalkyl, Trifluormethyl und Niederalkoxy steht; und $R_1$ und $R_2$ unabhängig voneinander jeweils für Niederalkyl oder unsubstituiertes oder durch Halogen, Trifluormethyl, Niederalkyl oder Niederalkoxy substituiertes Phenyl stehen, wobei auch einer der beiden Reste $R_1$ und $R_2$ für Wasserstoff stehen kann, oder gemeinsam eine Alkylenkette mit 2 bis 5 Kohlenstoffatomen bedeuten, welche unsubstituiert oder durch Niederalkyl substituiert ist;
oder Salzen davon, zur Herstellung von pharmazeutischen Präparaten zur Behandlung von Tumorerkrankungen und anderen proliferativen Erkrankungen; wobei das Präfix "Nieder" bedeutet, dass der betreffende Rest maximal bis und mit 7 Kohlenstoffatome hat.

**2.** Verwendung gemäss Anspruch 1, wobei es sich bei den zu behandelnden Erkrankungen um epidermale Hyperproliferation, Neoplasien epithelialen Charakters oder Leukämien handelt.

**3.** Verwendung gemäss einem der Ansprüche 1 bis 3, wobei die Verbindungen der Formel I aus solchen Verbindungen ausgewählt sind, worin n = 0 bis 2 ist, der Rest R für einen Substituenten ausgewählt aus Halogen, Niederalkoxy, Trifluormethyl und Niederalkyl steht; und $R_1$ und $R_2$ unabhängig voneinander jeweils für Niederalkyl oder unsubstituiertes oder durch Halogen, Trifluormethyl, Niederalkyl oder Niederalkoxy substituiertes Phenyl stehen, wobei einer der Reste $R_1$ und $R_2$ auch Wasserstoff bedeuten kann, oder gemeinsam eine Alkylenkette mit 4 bis 5 Kohlenstoffatomen bedeuten, welche unsubstituiert oder durch Niederalkyl substituiert ist; oder Salzen davon; wobei das Präfix "Nieder" bedeutet, dass der betreffende Rest maximal bis und mit 7 Kohlenstoffatome hat.

**4.** Eine Verbindung der in Anspruch 1 gezeigten Formel I, worin n = 1 oder 2 ist, R in m- (n = 1) oder m,m-Position (n = 2) gebunden ist und aus Fluor, Chlor, Brom und Niederalkoxy ausgewählt ist, und $R_1$ und $R_2$ unabhängig voneinander jeweils für Niederalkyl oder unsubstituiertes oder durch Halogen, Trifluormethyl, Niederalkyl oder

Niederalkoxy substituiertes Phenyl stehen, wobei auch einer der beiden Reste $R_1$ und $R_2$ für Wasserstoff stehen kann, oder gemeinsam eine Alkylenkette mit 2 bis 5 Kohlenstoffatomen bedeuten, welche unsubstituiert oder durch Niederalkyl substituiert ist; oder Salze davon; wobei das Präfix "Nieder" bedeutet, dass der betreffende Rest maximal bis und mit 7 Kohlenstoffatome hat.

**5.** Eine Verbindung der Formel I gemäss Anspruch 4, worin n gleich 1 ist; R für in m-Position gebundenes Chlor oder Brom steht, $R_1$ Wasserstoff bedeutet und $R_2$ 2-, 3- oder 4-Niederalkoxyphenyl oder Diniederalkoxyphenyl bedeutet; oder ein Salz davon; wobei das Präfix "Nieder" bedeutet, dass der betreffende Rest maximal bis und mit 7 Kohlenstoffatome hat.

**6.** Eine Verbindung gemäss Anspruch 4 der Formel I, worin n = 1 ist, R in m-Position gebunden ist und Brom oder insbesondere Chlor bedeutet; und $R_1$ und $R_2$ unabhängig voneinander für Niederalkyl oder ferner für Phenyl stehen, oder gemeinsam einen Tetramethylenrest bilden, oder Salze davon; wobei das Präfix "Nieder" bedeutet, dass der betreffende Rest maximal bis und mit 7 Kohlenstoffatome hat.

**7.** Eine Verbindung gemäss Anspruch 4 der Formel I, worin n = 1 ist, R in m-Position gebundenes Chlor oder Brom bedeutet und $R_1$ und $R_2$ jeweils Methyl bedeuten, oder Salze davon.

**8.** Eine Verbindung der Formel I gemäss Anspruch 4, ausgewählt aus

4-(m-Bromanilino)-5,6-dimethyl-7H-pyrrolo[2,3-d]pyrimidin,
4-(m-Fluoranilino)-5,6-dimethyl-7H-pyrrolo[2,3-d]pyrimidin,
4-(m,m-Dichloranilino)-5,6-dimethyl-7H-pyrrolo[2,3-d]pyrimidin,
4-(m-Methoxyanilino)-5,6-dimethyl-7H-pyrrolo[2,3-d]pyrimidin,
4-(m-Chloranilino)-5,6-diphenyl-7H-pyrrolo[2,3-d]pyrimidin,
4-(m-Bromanilino)-5,6-diphenyl-7H-pyrrolo[2,3-d]pyrimidin und
4-(m-Chloranilino)-5,6-tetramethylen-7H-pyrrolo[2,3-d]pyrimidin der Formel I, oder einem pharmazeutisch verwendbaren Salz davon.

**9.** Eine Verbindung der Formel I gemäss Anspruch 4 ausgewählt aus

4-(m-Bromanilino)-5,6-tetramethylen-7H-pyrrolo[2,3-d]pyrimidin,
4-(m-Fluoranilino)-5,6-tetramethylen-7H-pyrrolo[2,3-d]pyrimidin,
4-(m-Methoxyanilino)-5,6-tetramethylen-7H-pyrrolo[2,3-d]pyrimidin,
4-(m-Chloranilino)-6-(4-methoxyphenyl)-7H-pyrrolo[2,3-d]pyrimidin,
4-(m-Chloranilino)-6-(2,5-dimethoxyphenyl)-7H-pyrrolo[2,3-d]pyrimidin,
4-(m-Chloranilino)-6-(phenyl)-7H-pyrrolo [2,3-d]pyrimidin,
4-(m-Chloranilino)-5-methyl-6-(4-methoxyphenyl)-7H-pyrrolo[2,3-d]pyrimidin,
4-(m-Chloranilino)-6-(3-methoxyphenyl)-7H-pyrrolo[2,3-d]pyrimidin und
4-(m-Chloranilino)-6-(2-methoxyphenyl)-7H-pyrrolo[2,3-d]pyrim idin,

oder einem pharmazeutisch verwendbaren Salz davon.

**10.** 4-(m-Chloranilino)-5,6-dimethyl-7H-pyrrolo[2,3-d]pyrimidin der Formel I gemäss Anspruch 4, oder ein pharmazeutisch verwendbares Salz davon.

**11.** Eine Verbindung der Formel I, oder ein pharmazeutisch verwendbares Salz davon, gemäss einem der Ansprüche 4 bis 10 zur Anwendung in einem diagnostischen oder therapeutischen Verfahren zur Behandlung des menschlichen oder tierischen Körpers.

**12.** Ein pharmazeutisches Präparat, enthaltend eine Verbindung der Formel I, oder ein pharmazeutisch verwendbares Salz davon, gemäss einem der Ansprüche 4 bis 10 und mindestens ein pharmazeutisch verwendbares Trägermaterial.

**13.** Verfahren zur Herstellung einer Verbindung der Formel I gemäss Anspruch 10,
dadurch gekennzeichnet, dass man

a) ein Halogen-pyrrolo-pyrimidin der Formel II,

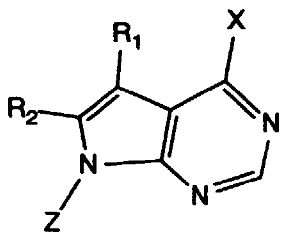

(II)

worin $R_1$ und $R_2$ jeweils Methyl bedeuten, Z für Wasserstoff oder für 1-Arylniederalkan-1-yl steht und X ein Halogenatom bedeutet, mit einem Phenylamin der Formel III,

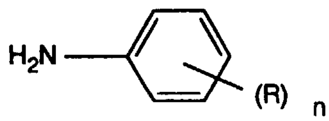

(III)

worin R für m-Chlor und n für 1 steht, umsetzt und, falls Z für 1-Aryl-niederalkan-1-yl steht, den Rest unter Desalkylierung abspaltet; oder

b) ein Pyrrolo-pyrimidinon der Formel IV,

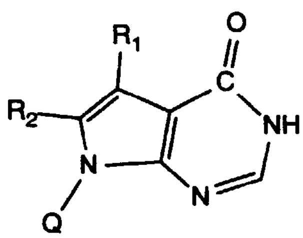

(IV)

worin $R_1$ und $R_2$ jeweils Methyl bedeuten und Q für Wasserstoff oder 1-Aryl-niederalkan-1-yl steht, in Gegenwart eines Dehydratisierungsmittels und eines tertiären Amins mit einem Phenylamin der Formel III, wie zuletzt genannt, umsetzt,

und gewünschtenfalls eine erhaltene freie Verbindung der Formel I in ein Salz umwandelt, und/oder ein erhaltenes Salz einer Verbindung der Formel I die freie Verbindung oder in ein anderes Salz umwandelt.

**Claims**

**1.** The use of a compound of formula I

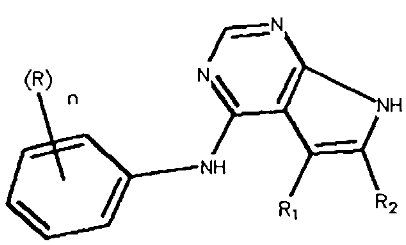

(I),

wherein

n is from 0 to 5 and
R is a substituent selected from halogen, lower alkyl, trifluoromethyl and lower alkoxy; and
$R_1$ and $R_2$ are each independently of the other lower alkyl, or phenyl that is unsubstituted or substituted by halogen, trifluoromethyl, lower alkyl or by lower alkoxy, it also being possible for one of the two radicals $R_1$ and $R_2$ to be hydrogen, or $R_1$ and $R_2$ together form an alkylene chain having from 2 to 5 carbon atoms that is unsubstituted or substituted by lower alkyl;

or of a salt thereof, in the preparation of a pharmaceutical composition for the treatment of tumour diseases and other proliferative diseases; the term "lower" indicating that the radical in question has up to and including a maximum of 7 carbon atoms.

**2.** The use according to claim 1, wherein the diseases to be treated are epidermal hyperproliferation, neoplasias of epithelial nature or leukaemias.

**3.** The use according to any one of claims 1 to 3, wherein the compound of formula I is selected from such compounds wherein n is from 0 to 2; the radical R is a substituent selected from halogen, lower alkoxy, trifluoromethyl and lower alkyl; and $R_1$ and $R_2$ are each independently of the other lower alkyl, or phenyl that is unsubstituted or substituted by halogen, trifluoromethyl, lower alkyl or by lower alkoxy, it also being possible for one of the radicals $R_1$ and $R_2$ to be hydrogen, or $R_1$ and $R_2$ together form an alkylene chain having 4 or 5 carbon atoms that is unsubstituted or substituted by lower alkyl; and salts thereof; the term "lower" indicating that the radical in question has up to and including a maximum of 7 carbon atoms.

**4.** A compound of formula I shown in claim 1, wherein n is 1 or 2, R is bonded in the m-position (n = 1) or in the m, m-position (n = 2) and is selected from fluorine, chlorine, bromine and lower alkoxy, and $R_1$ and $R_2$ are each independently of the other lower alkyl, or phenyl that is unsubstituted or substituted by halogen, trifluoromethyl, lower alkyl or by lower alkoxy, it also being possible for one of the two radicals $R_1$ and $R_2$ to be hydrogen, or $R_1$ and $R_2$ together form an alkylene chain having from 2 to 5 carbon atoms that is unsubstituted or substituted by lower alkyl; or a salt thereof; the term "lower" indicating that the radical in question has up to and including a maximum of 7 carbon atoms.

**5.** A compound of formula I according to claim 4, wherein n is 1; R is chlorine or bromine each bonded in the m-position; $R_1$ is hydrogen; and $R_2$ is 2-, 3- or 4-lower alkoxyphenyl or di-lower alkoxyphenyl; or a salt thereof; the term "lower" indicating that the radical in question has up to and including a maximum of 7 carbon atoms.

**6.** A compound according to claim 4 of formula I, wherein n is 1, R is bonded in the m-position and is bromine or especially chlorine; and $R_1$ and $R_2$ are each independently of the other lower alkyl or, further, phenyl, or $R_1$ and $R_2$ together form a tetramethylene radical, or a salt thereof; the term "lower" indicating that the radical in question has up to and including a maximum of 7 carbon atoms.

**7.** A compound according to claim 4 of formula I, wherein n is 1, R is chlorine or bromine each bonded in the m-position, and $R_1$ and $R_2$ are each methyl, or a salt thereof.

**8.** A compound of formula I according to claim 4, selected from

4-(m-bromoanilino)-5,6-dimethyl-7H-pyrrolo[2,3-d]pyrimidine,
4-(m-fluoroanilino)-5,6-dimethyl-7H-pyrrolo[2,3-d]pyrimidine,
4-(m,m-dichloroanilino)-5,6-dimethyl-7H-pyrrolo[2,3-d]pyrimidine,
4-(m-methoxyanilino)-5,6-dimethyl-7H-pyrrolo[2,3-d]pyrimidine,
4-(m-chloroanilino)-5,6-diphenyl-7H-pyrrolo[2,3-d]pyrimidine,
4-(m-bromoanilino)-5,6-diphenyl-7H-pyrrolo[2,3-d]pyrimidine, and
4-(m-chloroanilino)-5,6-tetramethylene-7H-pyrrolo[2,3-d]pyrimidine of formula I, or a pharmaceutically acceptable salt thereof.

**9.** A compound of formula I according to claim 4, selected from

4-(m-bromoanilino)-5,6-tetramethylene-7H-pyrrolo[2,3-d]pyrimidine,

4-(m-fluoroanilino)-5,6-tetramethylene-7H-pyrrolo[2,3-d]pyrimidine,
4-(m-methoxyanilino)-5,6-tetramethylene-7H-pyrrolo[2,3-d]pyrimidine,
4-(m-chloroanilino)-6-(4-methoxyphenyl)-7H-pyrrolo[2,3-d]pyrimidine,
4-(m-chloroanilino)-6-(2,5-dimethoxyphenyl)-7H-pyrrolo[2,3-d]pyrimidine,
4-(m-chloroanilino)-6-(phenyl)-7H-pyrrolo[2,3-d]pyrimidine,
4-(m-chloroanilino)-5-methyl-6-(4-methoxyphenyl)-7H-pyrrolo[2,3-d]pyrimidine,
4-(m-chloroanilino)-6-(3-methoxyphenyl)-7H-pyrrolo[2,3-d]pyrimidine, and
4-(m-chloroanilino)-6-(2-methoxyphenyl)-7H-pyrrolo[2,3-d]pyrimidine,

or a pharmaceutically acceptable salt thereof.

**10.** 4-(m-Chloroanilino)-5,6-dimethyl-7H-pyrrolo[2,3-d]pyrimidine of formula I according to claim 4, or a pharmaceutically acceptable salt thereof.

**11.** A compound of formula I, or a pharmaceutically acceptable salt thereof, according to any one of claims 4 to 10 for use in a diagnostic or therapeutic method for the treatment of the human or animal body.

**12.** A pharmaceutical composition comprising a compound of formula I, or a pharmaceutically acceptable salt thereof, according to any one of claims 4 to 10 and at least one pharmaceutically acceptable carrier.

**13.** A process for the preparation of a compound of formula I according to claim 10, which comprises

a) reacting a halo-pyrrolo-pyrimidine of formula II

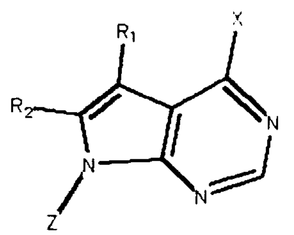

(II),

wherein $R_1$ and $R_2$ are each methyl, Z is hydrogen or 1-aryllower alkan-1-yl and X is a halogen atom, with a phenylamine of formula III

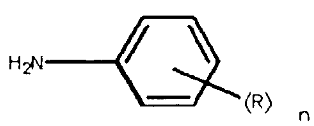

(III),

wherein R is m-chlorine and n is 1, and, when Z is 1-aryllower alkan-1-yl, removing the radical with dealkylation; or

b) reacting a pyrrolo-pyrimidinone of formula IV

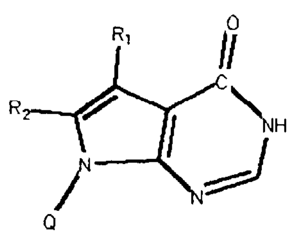

(IV),

wherein $R_1$ and $R_2$ are each methyl and Q is hydrogen or 1-aryl-lower alkan-1-yl, with a phenylamine of formula III as mentioned above, in the presence of a dehydrating agent and a tertiary amine,

and, if desired, converting a resulting free compound of formula I into a salt and/or converting a resulting salt of a compound of formula I into the free compound or into a different salt.

## Revendications

**1.** Utilisation de composés de formule I :

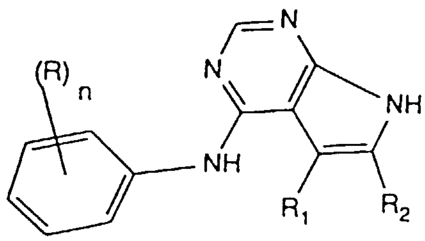

(I)

dans laquelle :

n vaut de 0 à 5, et R représente un ou plusieurs substituants choisis parmi les atomes d'halogène, les groupes alkyle inférieur, le groupe trifluorométhyle et les groupes alcoxy inférieur ; et $R_1$ et $R_2$, indépendamment l'un de l'autre, désignent chacun un groupe alkyle inférieur, ou phényle non substitué ou substitué par des substituants halogéno, trifluorométhyle, alkyle inférieur ou alcoxy inférieur, auquel cas aussi l'un des deux radicaux $R_1$ et $R_2$ peut être un hydrogène, ou encore représentent ensemble une chaîne alkylène ayant de 2 à 5 atomes de carbone, qui est non substituée ou substituée par des substituants alkyle inférieur ; ou leurs sels, pour fabriquer des préparations pharmaceutiques destinées au traitement de maladies tumorales et d'autres maladies prolifératives ; où l'adjectif "inférieur" signifie que le radical correspondant a un nombre d'atomes de carbone inférieur ou égal à 7.

**2.** Utilisation selon la revendication 1, dans laquelle, pour ce qui est des maladies à traiter, il s'agit de l'hyperprolifération épidermique, des néoplasies de caractère épithélial ou de leucémies.

**3.** Utilisation selon l'une des revendications 1 à 3, dans laquelle les composés de formule I sont choisis parmi les composés dans lesquels n = 0 à 2, le radical R est un substituant choisi parmi les radicaux halogéno, alcoxy inférieur, trifluorméthyle et alkyle inférieur ; et $R_1$ et $R_2$, indépendamment l'un de l'autre, sont chacun un radical alkyle inférieur, ou phényle non substitué ou substitué par des substituants halogéno, trifluorométhyle, alkyle inférieur ou alcoxy inférieur, où l'un des radicaux $R_1$ et $R_2$ peut aussi être un hydrogène, ou encore ils forment ensemble une chaîne alkylène ayant de 4 à 5 atomes de carbone, qui est non substituée ou substituée par des groupes alkyle inférieur ; ou leurs sels ; où l'adjectif "inférieur" signifie que le radical correspondant contient un nombre d'atomes de carbone inférieur ou égal à 7.

**4.** Composé ayant la formule I selon la revendication 1, dans laquelle n = 1 ou 2, R est fixé en position m (n = 1) ou en position m,m (n = 2) et est choisi parmi les radicaux fluoro, chloro, bromo et alcoxy inférieur, et $R_1$ et $R_2$, indépendamment l'un de l'autre, sont chacun un radical alkyle inférieur ou phényle non substitué ou substitué par des substituants halogéno, trifluorométhyle, alkyle inférieur ou alcoxy inférieur, où aussi l'un des deux radicaux $R_1$ et $R_2$ peut être un hydrogène, ou encore ils forment ensemble une chaîne alkylène ayant de 2 à 5 atomes de carbone, qui est non substituée ou substituée par des groupes alkyle inférieurs ; ou ses sels ; où l'adjectif "inférieur" signifie que le radical correspondant a un nombre d'atomes de carbone inférieur ou égal à 7.

**5.** Composé de formule I selon la revendication 4, dans laquelle n vaut 1 ; R est un atome de chlore ou de brome fixé en position m, $R_1$ est un hydrogène, et $R_2$ est un radical 2-, 3- ou 4-(alcoxy inférieur)phényle ou di(alcoxy inférieur)phényle ; ou l'un de ses sels ; et où l'adjectif "inférieur" signifie que le radical correspondant a un nombre d'atomes de carbone inférieur ou égal à 7.

**6.** Composé de formule I selon la revendication 4, dans laquelle n = 1, R est fixé en position m et est un atome de brome ou en particulier de chlore ; et $R_1$ et $R_2$, indépendamment l'un de l'autre, sont des groupes alkyle inférieurs

ou de plus phényle, ou forment ensemble un radical tétraméthylène, ou ses sels ; où l'adjectif "inférieur" signifie que le radical correspondant a un nombre d'atomes de carbone inférieur ou égal à 7.

**7.** Composé de formule I selon la revendication 4, dans laquelle n = 1, R est un atome de chlore ou de brome fixé en position m, et $R_1$ et $R_2$ représentent chacun le groupe méthyle, ou ses sels.

**8.** Composé de formule I selon la revendication 4, choisi parmi les composés suivants :

4-(m-bromanilino)-5,6-diméthyl-7H-pyrrolo[2,3-d]pyrimidine,
4-(m-fluoranilino)-5,6-diméthyl-7H-pyrrolo[2,3-d]pyrimidine,
4-(m,m-dichloranilino)-5,6-diméthyl-7H-pyrrolo[2,3-d]pyrimidine,
4-(m-méthoxyanilino)-5,6-diméthyl-7H-pyrrolo[2,3-d]pyrimidine,
4-(m-chloranilino)-5,6-tétraméthylène-7H-pyrrolo[2,3-d]pyrimidine,
4-(m-bromanilino)-5,6-tétraméthylène-7H-pyrrolo[2,3-d]pyrimidine,
4-(m-fluoranilino)-5,6-tétraméthylène-7H-pyrrolo[2,3-d]pyrimidine, ou un de ses sels utilisables d'un point de vue pharmaceutique.

**9.** Composé de formule I selon la revendication 4, choisi parmi les composés suivants :

4-(m-bromanilino)-5,6-tétraméthylène-7H-pyrrolo[2,3-d]-pyrimidine,
4-(m-fluoranilino)-5,6-tétraméthylène-7H-pyrrolo[2,3-d]-pyrimidine,
4-(m-méthoxyanilino)-5,6-tétraméthylène-7H-pyrrolo[2,3-d]-pyrimidine,
4-(m-chloranilino)-6-(4-méthoxyphényl)-7H-pyrrolo[2,3-d]-pyrimidine,
4-(m-chloranilino)-6-(2,5-diméthoxyphényl)-7H-pyrrolo[2,3-d]-pyrimidine,
4-(m-chloranilino)-6-(phényl)-7H-pyrrolo[2,3-d]pyrimidine,
4-(m-chloranilino)-5-méthyl-6-(4-méthoxyphényl)-7H-pyrrolo[2,3-d]-pyrimidine,
4-(m-chloranilino)-6-(3-méthoxyphényl)-7H-pyrrolo[2,3-d]-pyrimidine, et
4-(m-chloranilino)-6-(2-méthoxyphényl)-7H-pyrrolo[2,3-d]-pyrimidine, ou l'un de ses sels utilisables d'un point de vue pharmaceutique.

**10.** 4-(m-Chloranilino)-5,6-diméthyl-7H-pyrrolo[2,3-d]-pyrimidine de formule I selon la revendication 4, ou l'un de ses sels utilisables d'un point de vue pharmaceutique.

**11.** Composé de formule I, ou l'un de ses sels utilisables d'un point de vue pharmaceutique, selon l'un des revendications 4 à 10, pour utilisation dans un procédé diagnostique ou thérapeutique destiné au traitement de l'organisme humain ou animal.

**12.** Préparation pharmaceutique contenant un composé de formule I ou un sel utilisable d'un point de vue pharmaceutique de ce composé, selon l'une des revendications 4 à 10, et au moins un excipient utilisable d'un point de vue pharmaceutique.

**13.** Procédé de préparation d'un composé de formule I selon la revendication 10, caractérisé en ce que :

a) on fait réagir une halogénopyrrolo-pyrimidine de formule II :

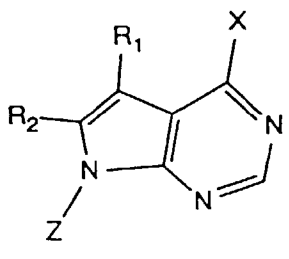

(II)

dans laquelle :

$R_1$ et $R_2$ sont chacun des groupes méthyle, Z est un hydrogène ou un groupe 1-aryl-(alcane inférieur)-1-yle et X est un atome d'halogène, avec une phénylamine de formule III :

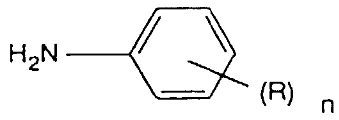

(III)

dans laquelle :

R est le radical m-chloro et n vaut 1, et, si Z est un groupe 1-aryl-(alcane inférieur)-1-yle, on dissocie le radical par désalkylation ; ou bien

b) on fait réagir une pyrrolo-pyrimidinone de formule IV :

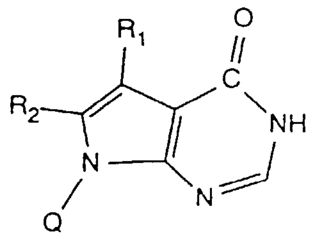

(IV)

dans laquelle :

$R_1$ et $R_2$ sont chacun le groupe méthyle et Q est un hydrogène ou un groupe 1-aryl-(alcane inférieur)-1-yle, en présence d'un agent de déshydratation et d'une amine tertiaire, avec une phénylamine de formule III telle que mentionnée en dernier,

et, si on le souhaite, on convertit en un sel un composé libre tel qu'obtenu de formule I, et/ou on convertit en le composé libre ou en un autre sel un sel tel qu'obtenu d'un composé de formule I.